# EUROPÄISCHE PATENTSCHRIFT

(11) **EP 4 345 754 B1**
(45) Veröffentlichungstag und Bekanntmachung des Hinweises auf die Patenterteilung: **02.09.2026**
(21) Anmeldenummer: 22198051.9
(22) Anmeldetag: 27.09.2022
(51) Int. Cl.: G06T 11/60

(54) **VERFAHREN UND SYSTEM ZUR UNTERSTÜTZUNG EINER AUSWERTUNG EINER VIDEOUNTERSTÜTZTEN MEDIZINISCHEN EINGRIFFSPROZEDUR**
METHOD AND SYSTEM FOR ASSISTING EVALUATION OF A VIDEO-ASSISTED MEDICAL INTERVENTION PROCEDURE
PROCÉDÉ ET SYSTÈME POUR PRENDRE EN CHARGE UNE ÉVALUATION D'UNE PROCÉDURE MÉDICALE ASSISTÉE PAR VIDÉO

(43) Veröffentlichungstag der Anmeldung: 03.04.2024
(73) Patentinhaber: Erbe Elektromedizin GmbH, 72072 Tübingen (DE)
(72) Erfinder: SELIG, Peter, 72147 Nehren (DE)
(74) Vertreter: Rüger Abel IP Legal Solutions GmbH

(56) Entgegenhaltungen:
- DE-A1- 102017 105 169
- US-A1- 2022 233 229
- UNTERWEGER ANDREAS: "Compression artifacts in modern video coding and state-of-the-art means of compensation", 1 January 2012 (2012-01-01), pages 1 - 21, XP055981867, Retrieved from the Internet <URL:https://wavelab.at/papers/Unterweger13a.pdf> [retrieved on 20211116]

## Beschreibung

Die vorliegende Erfindung betrifft ein System und computer-implementiertes Verfahren zur Unterstützung einer Auswertung einer videounterstützten medizinischen Eingriffsprozedur. Insbesondere betrifft die Erfindung derartige Verfahren und Systeme, die zum simultanen Aufzeichnen von Videobilddaten von einem medizinischen Eingriff und weiteren mit dem Eingriff verbundenen Daten, einschließlich Messdaten, in der Lage sind, um die Videobild- und weiteren Daten nachträglich auswerten zu können.

Während eines videounterstützten medizinischen Eingriffs gesammelte Bilddaten können viele wertvolle Informationen bspw. für den Patienten oder den behandelnden Arzt liefern. Sie können zum Qualitätsnachweis und zur Qualitätssicherung der vorgenommenen Behandlung oder Untersuchung, einschließlich für die Erforschung, Dokumentation und Auswertung der durchgeführten Maßnahmen, dienen, dem Patienten das Verständnis der vorgenommenen Behandlung oder Untersuchung erleichtern und als Grundlage für eine operative Nachsorge einbezogen werden. Bei derartigen Eingriffen können auch weitere Informationen für die Auswertung einer videounterstützten medizinischen Eingriffsprozedur von Interesse sein, zu denen bspw. Informationen, die die Behandlung oder Untersuchung als solche, ihre Art, den Patienten oder den behandelnden Arzt, betreffen, Informationen, die die eingesetzten medizinischen Geräte betreffen, während des medizinischen Eingriffs gewonnene Diagnose- und/oder Messdaten gehören können. All diese zusätzlichen Informationen können für die nachträgliche Überprüfung, Nachvollziehbarkeit und Auswertung der durchgeführten medizinischen Behandlungen und/oder Untersuchungen in Verbindung mit den aufgenommenen Videodaten weitere Erkenntnisse von hohem Wert liefern.

Eine Problematik in diesem Zusammenhang betrifft die geeignete Speicherung all dieser Informationen bzw. Daten. Momentan gibt es in der Praxis keine geeigneten Verfahren und Systeme, die eine geeignete Speicherung der Videobilddaten mit zeitlicher Verknüpfung der weiteren Daten für eine effiziente nachträgliche Auswertung einer videounterstützten medizinischen Eingriffsprozedur in effizienter Weise ermöglichen würden. In der Praxis werden im Allgemeinen für die Speicherung von Videos in den Krankenhäusern etablierte Systeme verwendet, während solche Systeme für Geräte-, Diagnose- oder Messdaten in der Krankenhausumgebung fehlen. Gerätedaten können manchmal in separaten Systemen gespeichert werden, die kein Standardsystem in einem Krankenhaus sind und sich auch nicht in direktem Zugriffsbereich des Krankenhauses befinden, sondern bspw. bei dem Hersteller des jeweiligen medizinischen Gerätes untergebracht sind.

Wenn Daten aus zwei verschiedenen Systemen zusammengeführt werden müssen, ist die Frage der zeitlichen Zuordnung und Synchronisation kritisch. Eine exakte Synchronisation von bspw. elektrischen Daten aus einer HF-Chirurgie zu einzelnen Videoframes der aufgenommenen Videobilddaten ist praktisch nicht möglich. Außerdem erschweren getrennte Systeme die Einhaltung von Datenschutz und Datensicherheit.

Aus der DE 10 2014 210 079 A1 ist ein System zur Dokumentation von Operationen bekannt, in dem medizinisches Bildmaterial mit einem Sicherheitsmerkmal, z.B. einem digitalen Wasserzeichen, versehen wird, das in die Bilddaten integriert wird, ohne sichtbar zu sein. Auf diese Weise können auch weitere statische Informationen, wie bspw. Typ, Seriennummer, des medizinischen Gerätes, Zertifikate, Name des Patienten und des behandelnden Arztes, und dgl., mit in die Bilddaten integriert werden.

US 6 104 948 A offenbart ein Verfahren und System zur visuellen Integration mehrerer Datenakquisitionstechnologien für eine Echtzeit-Analyse, wobei Daten aus verschiedenen Quellen, wie bspw. Datum, Zeit, elektrophysiologische Daten und Videodaten, in einem Computer miteinander kombiniert und gemeinsam, nebeneinander auf einem Monitor angezeigt werden. Das auf dem Monitor angezeigte kombinierte Bild kann anschließend abgespeichert werden.

WO 2012/162427 A2 offenbart ein allgemeines System zur Einbettung von Metadaten in Videoinhalte und Fernsehübertragungen. Die Metadaten werden in Form von QR-Codes in die übertragenen Video- bzw. Fernsehbilder eingebettet, um einem Zuschauer zu ermöglichen, schnell und vorzugsweise automatisiert eine in den Metadaten enthaltene Zeichenfolge, wie bspw. einen Befehlssatz oder Link, zu dekodieren.

EP 2 063 374 A1 offenbart ein Verfahren und eine Vorrichtung zur Dokumentation medizinischer Daten, wobei zusätzlich zu Videodaten, z.B. endoskopischen Bilddaten, auch weitere Informationen, einschließlich Daten über Art, Ort, Funktion und/oder Parameter der verwendeten Instrumente bzw. Geräte, Daten über Druck und Fluss eines Insufflationsgases und damit zusammenhängende Messwerte, z.B. Druck, Leckstrom, Temperatur, oder auch Anästhesiedaten und Vitalparameter gespeichert werden können, um eine möglichst vollständige Dokumentation der Vorgänge bei einer Operation zu ermöglichen. Um die Menge der aufgenommenen Daten zu begrenzen, wird die Speicherung nur bei Erfassung der Präsenz eines zu operierenden Patienten in dem Operationssaal aktiviert.

WO 03/001359 A2 offenbart ein System zum simultanen Aufzeichnen von Messdaten und Video-/Audiodaten insbesondere zur Dokumentation von psychologischen Therapiesitzungen, wobei neben Videodaten z.B. die Herzfrequenz aufgenommen wird. Es können auch Operations- und diagnostische Daten mit dokumentiert werden. Zum simultanen Aufzeichnen der Messdaten und Video- und/oder Audiodaten werden sowohl die Messdaten als auch die Video- und/oder Audiodaten jeweils in Datenpakete gleicher Paketlänge zerlegt und in einer geordneten Weise in einer gemeinsamen Datendatei abgelegt. Die Video- und/oder Audiodaten werden zuerst komprimiert, in Pakete zerlegt und mit den Paketen der Messdaten kombiniert.

WO 2006/131277 A1 offenbart ein System zur Befundung, Kommentierung und/oder Dokumentation von Bewegtbildern im medizinischen Bereich, wie z.B. Ultraschallbildern oder endoskopischen Videoaufnahmen. In die Bilder bzw. Videos werden Annotierungen, wie z.B. Markierungen, Kommentare, Bilder, Text, Audio, etc., aufgenommen und beim erneuten Abspielen des Videos an der passenden Stelle mit abgespielt.

WO 2014/181230 A2 offenbart ein Verfahren zum Einbetten von kodierten Informationen, z.B. Bildparametern und Diagnoseinformationen, in medizinische Bilder. Die Informationen werden in die Bilddaten eingebrannt oder auf einer gesonderten Schicht, bspw. einer DICOM-Überlagerungsschicht, über den Bilddaten gespeichert. In Ausführungsformen werden die Informationen in Form eines Barcodes oder QR-Codes in einem Bereich eines Bildes außerhalb der visualisierten Anatomie oder entlang der Außenränder des Bildes positioniert.

EP 3 836 155 A1 offenbart die Integration von Informationen, z.B. Patientendaten, Metadaten oder aus Deep Learning oder neuronalen Netzwerken extrahierten Merkmalen in medizinische diagnostische Bilder. Die Informationen können in Barcodes oder QR-Codes codiert, den Bildern überlagert oder mit den Bildern transparent verschmolzen werden, und sie werden gemeinsam mit den Bildern oder abwechselnd zu diesen angezeigt.

DE 10 2017 105 169 A1 offenbart ein computer-implementiertes Verfahren zur Unterstützung einer Auswertung einer videounterstützten medizinischen Eingriffsprozedur, das die Merkmale des Oberbegriffs des Patentanspruchs 1 aufweist, und ein computerbasiertes System zur Ausführung eines derartigen Verfahrens.

Die vorstehend erwähnten Systeme und Verfahren nach dem Stand der Technik leiden an verschiedenen Unzulänglichkeiten. Entweder ermöglichen sie keine synchrone Zuordnung von weiteren Daten, wie Untersuchungs-, Geräte- und Messdaten, zu aufgenommenen Videobilddaten, oder sie erfordern einen relativ hohen Aufwand, um eine zeitsynchrone Speicherung und Synchronisierung der Video- und weiteren Daten sicherzustellen.

Außerdem sind den Systemen und Verfahren Grenzen hinsichtlich der Auswertungsmöglichkeiten gesetzt. So können z.B. in der Hochfrequenz(HF)-Chirurgie die Zusammenhänge zwischen den Behandlungseffekten und den eingestellten Größen, Parametern und gewählten Anwendungsmodi, die Wirksamkeiten der angestrebten Behandlungseffekte und dgl. anhand der aufgenommenen Videobilddaten und der mit abgespeicherten statischen Behandlungs- und Gerätedaten nur unzureichend bewertet werden. Insbesondere sind viele Vorgänge, die z.B. die Funkenbildung an dem HF-Chirurgieinstrument, die Einleitung eines hochfrequenten Wechselstroms durch den menschlichen Körper und dessen Wechselwirkung mit dem behandelten Gewebe, die damit verursachte Erwärmung des Gewebes und die resultierenden Schneid- oder Koagulationseffekte betreffen, sehr schnelle und zeitkritische Vorgänge, die so nicht leicht zu beurteilen sind. Es bleibt weiterhin ein Bedarf nach Systemen und Verfahren, die die Unzulänglichkeiten von herkömmlichen Systemen und Verfahren beseitigen.

Ausgehend hiervon ist es eine Aufgabe der vorliegenden Erfindung, ein computer-implementiertes Verfahren und System zur Unterstützung einer Auswertung einer videounterstützten medizinischen Eingriffsprozedur zu schaffen, die eine zeitliche Zuordnung und Synchronisation von Videobilddaten und weiteren Daten bei relativ geringem Aufwand für die Speicherung der Daten ermöglichen. Insbesondere sollten das System und das computer-implementierte Verfahren eine zuverlässige nachträgliche Beurteilung der medizinischen Eingriffsprozedur, einschließlich zeitkritischer Vorgänge, insbesondere eines videounterstützten HF-Chirurgieeingriffs, ermöglichen.

Diese Aufgabe wird durch das computer-implementierte Verfahren und das System zur Unterstützung einer Auswertung einer videounterstützten medizinischen Eingriffsprozedur, die die Merkmale der unabhängigen Patentansprüche 1 und 14 aufweisen, gelöst. Besonders bevorzugte Ausführungsformen der Erfindung sind Gegenstand der abhängigen Patentansprüche.

Gemäß einem Aspekt der vorliegenden Erfindung ist ein computer-implementiertes Verfahren zur Unterstützung einer Auswertung einer videounterstützten medizinischen Eingriffsprozedur geschaffen, wobei das computer-implementierte Verfahren die Schritte aufweist: Empfangen von medizinischen Videobilddaten, die während eines medizinischen Eingriffs von einer Videokamera mit einer bestimmten Framerate aufgenommene Videobilder einer untersuchten oder behandelten Anatomie repräsentieren; Empfangen von weiteren Daten, die wenigstens eines von Behandlungs- oder Untersuchungs-, Geräte-, Diagnose- und Messdaten im Zusammenhang mit dem medizinischen Eingriff umfassen, wobei zu den weiteren Daten gemessene dynamische Daten gehören, die sich während einer Frame-Dauer verändern; Identifizieren von Zielbereichen in den Videobildern zur Einbettung der weiteren Daten; und Modifizieren der Videobilddaten durch zeitlich synchrones Einbetten der weiteren Daten in die Videobilddaten, indem Videobilddaten in den identifizierten Zielbereichen eines Frames durch die zu dem jeweiligen Frame zugehörigen weiteren Daten ersetzt werden. Zu den gemessenen dynamischen Daten gehören Messgrößen, die mit einer Aktualisierungsrate erfasst oder ermittelt werden, die um ein Mehrfaches größer ist als die Framerate der Videobilddaten, wobei die Aktualisierungsrate wenigstens 150 Hz beträgt. Dabei werden in einzelnen Frames gemessene dynamische Daten gespeichert, die einen zeitlichen Verlauf von zwei oder mehreren Messwerten einer Messgröße kennzeichnen.

Das erfindungsgemäße computer-implementierte Verfahren (nachfolgend kurz als Verfahren bezeichnet) ermöglicht ein zeitsynchrones Aufzeichnen von Videobilddaten und zugehörigen weiteren Informationen, die von einem videounterstützten medizinischen Eingriff, einer medizinischen Behandlung, Untersuchung oder Operation, herrühren. Das Verfahren wird vorzugsweise online, parallel zu der Durchführung des medizinischen Eingriffs, und in Echtzeit oder zumindest nahezu in Echtzeit ausgeführt. Der medizinische Eingriff kann insbesondere ein HF-Elektrochirurgieeingriff, wie bspw. eine Koagulation, Thermofusion, HF-Schneiden (Elektrotomie), und/oder eine Untersuchung, wie bspw. eine optische Emissionsspektrometrie- oder Impedanzspektroskopie-Untersuchung sein. Die Informationen können auch eine Therapie, wie **z.B.** HF-, Kryo- oder Wasserstrahltherapie und damit verbundene Geräte sowie weitere Systemkomponenten, wie bspw. Einrichtungen zum Rauchabzug, und/oder andere Geräte, Instrumente und Einrichtungen in einem Operationssaal, wie **z.B.** den Patientenmonitor, einen Operationstisch und dgl., betreffen, die alle über eine Datenschnittstelle miteinander vernetzt sein können, so dass deren Daten mit erfasst und in den Videobilddaten gespeichert werden können.

Durch die direkte Einbettung der Behandlungs- oder Untersuchungs-, Geräte-, Diagnose- und/oder Messdaten unmittelbar in die Videobilder, die den Fortgang eines videounterstützten medizinischen Eingriffs dokumentieren, kann bei reduziertem Speicheraufwand eine hohe Zeitsynchronität zwischen den Video- und weiteren Daten sichergestellt und eine äußerst einfache und effektive Auswertung aller Daten ermöglicht werden. Insbesondere können die gemessenen dynamischen Daten, **z.B.** die Messwerte von elektrischen HF-Größen, die sich mit sehr hoher Frequenz und insbesondere innerhalb eines jeden Frames verändern, in dem richtigen zugehörigen Frame mit abgelegt werden, was die Basis für eine effektive Auswertung auch zeitkritischer Vorgänge bspw. eines HF-Chirurgieeingriffs liefert. Z.B. kann so eine gute Beurteilung einer Funkenbildung, einschließlich des Zündverhaltens, der Funkenintensität oder eines Funkenabrisses, in einer HF-Schneid- oder HF-Koagulationsprozedur ermöglicht werden.

Die resultierenden modifizierten Videobilddaten können in einer nicht-flüchtigen Speichervorrichtung für eine weitere Verwendung, insbesondere zur Auswertung, gespeichert werden. Die Speichervorrichtung kann insbesondere in der Umgebung des Ortes, an dem die medizinische Eingriffsprozedur vorgenommen wurde, z.B. dem jeweiligen Krankenhaus, angeordnet sein. Als Speichervorrichtung kann ein Speicher in einem Computer oder einem Computersystem oder einem Server oder auch ein Cloud-Speicher dienen.

In einer besonders bevorzugten Ausführungsform werden die modifizierten Videobilddaten alternativ oder zusätzlich zu einem Bildarchivierungs- und Kommunikationssystem, sogenanntem Picture Archiving and Communication System (PACS), einem Krankenhausinformationssystem (KIS), auch als Hospital Information System (HIS) bezeichnet, oder einem externen Server zur Archivierung von Bild-/Videodaten übertragen, auf das oder den das Krankenhaus, in dem der medizinische Eingriff vorgenommen wurde, und/oder der Hersteller des verwendeten medizinischen Gerätes für Auswertezwecke zugreifen kann.

Die für die Videoaufnahme des medizinischen Eingriffs vorgesehene Videokamera liefert die Einzelbilder mit einer bestimmten Framerate, auch Bildwiederholrate genannt, die einer bestimmten Anzahl von Frames (Einzelbildern) pro Sekunde entspricht. Die Framerate kann zwischen 24 und 60 Frames pro Sekunde (fps) betragen. Bevorzugterweise beträgt sie 30, 50 oder 60 fps. Höhere Frameraten, wie 50 oder 60 fps, werden mehr bevorzugt, weil sich dann auch sehr zeitkritische Vorgänge, wie sie in der HF-Chirurgie von Interesse sind, optisch hoch auflösen lassen. Allerdings steigt bei höherer Framerate auch der Speicherbedarf.

Zu den Behandlungs- oder Untersuchungsdaten können z.B. Ort und Zeit des Eingriffs, die Institution, beteiligte Personen, wie z.B. der Name des Patienten, Name des behandelnden Arztes, die Art des medizinischen Eingriffs und/oder andere den medizinischen Eingriff betreffende Daten gehören.

Zu den Gerätedaten können z.B. eine Typenbezeichnung und Seriennummer des oder der verwendeten medizinischen Geräte, Geräteparameter, Standardeinstellungen, gewählter Anwendungsmode, vorgegebene Grenzwerte für Variablen, Fehler- und Statusmeldungen der Geräte und/oder andere die verwendeten medizinischen Geräte betreffenden Daten gehören.

Zu den Diagnosedaten können **z.B.** ermittelte Spektren und/oder Klassifikationsergebnisse aus einer optischen Emissionsspektrometrie, Spektren, Messwerte und/oder Klassifikationsergebnisse aus einer Impedanzspektroskopie oder aus anderen diagnostischen Verfahren und weitere Diagnosedaten gehören.

Zu den Messdaten, insbesondere den dynamischen Messdaten, können während einer HF-Eingriffsprozedur gemessene oder ermittelte elektrische HF-Größen, wie Spannung, Strom, Leistung, Leistungsfaktor und/oder Funkenintensität, z.B. bei HF-Schneid- und Koagulationsprozeduren, Parameter oder Größen einer Neutralelektrode bei Durchführung monopolarer HF-Techniken, wie z.B. Übergangsimpedanz, Stromsymmetrie und/oder Stromdichte, und/oder andere während des medizinischen Eingriffs gewonnene dynamische Messgrößen gehören.

Anstelle dieser Daten im Rohformat in dem Videobild zu integrieren, können die Daten vor der Einfügung ins Videobild verschlüsselt und/oder mit einem digitalen Zertifikat zur Sicherstellung der Authentizität versehen und/oder komprimiert werden, um Speicherplatz zu sparen.

Erfindungsgemäß gehören zu den gemessenen dynamischen Daten Messgrößen, die mit einer Aktualisierungsrate erfasst oder ermittelt werden, die um ein Mehrfaches, wenigstens Zweifaches oder Vielfaches, größer ist als die Framerate der Videobilddaten. Die Aktualisierungsrate beträgt wenigstens 150 Hz und kann vorzugsweise 250 Hz oder sogar noch mehr betragen. Somit können mehrere Messwerte einer dynamischen Messgröße pro Frame erfasst und gespeichert werden.

Ferner werden gemäß der Erfindung vorteilhafterweise in einzelnen Frames gemessene Daten gespeichert, die einen zeitlichen Verlauf von zwei oder mehreren Messwerten einer Messgröße kennzeichnen. Bei einer Framerate von bspw. 50 fps und einer Aktualisierungsrate von 250 Hz können bspw. fünf Messwerte im zeitlichen Verlauf zeitsynchron pro Frame der Videobilddaten gespeichert werden. Die Messdaten, die zu einem einzelnen Frame gehören, werden zeitsynchron gemeinsam mit den Videobilddaten abgespeichert, so dass eine feste zeitliche Zuordnung zwischen den Bildinformationen in dem einzelnen Frame und dem zugehörigen zeitlichen Verlauf der Messgröße gegeben ist.

Das Identifizieren von Zielbereichen in den Videobildern zur Einbettung der weiteren Daten kann einfach anhand im Voraus festgelegter oder durch einen Benutzer vorgegebener oder vorgebbarer geeigneter Bereiche in den Videobildern erfolgen. Z.B. können die Daten-Pixel der weiteren Daten zusammenhängend gespeichert werden und bspw. als ein kleiner Streifen oder Block an einer definierten Position in dem Bild abgelegt werden. Bevorzugterweise werden sie an mehreren definierten Positionen, über das Bild verteilt gespeichert.

Besonders bevorzugt erfolgt die Speicherung in weniger relevanten Bildbereichen, d.h. in Bildbereichen, die keinen oder einen nur geringen Nutzinformationsgehalt der Bilddaten aufweisen. Dies können z.B. Bereiche, die für die Beschriftung, angezeigte Patientendaten, Uhrzeit und dgl. reserviert sind, oder auch die Außenbildränder der Videobilder sein. Dies kann auch bevorzugterweise, sofern vorhanden, auch ein nicht sichtbarer Bereich, der zur Darstellung abgeschnitten wird.

Anstatt einzelne Pixel der Videobilddaten vollständig für die Datenspeicherung zu nutzen, kann in einer alternativen Speicherungsprozedur eine Überlagerung der Bilddaten mit der originalen Video-Pixelfarbe angewendet werden. In anderen Worten können die weiteren Daten in einzelne Bits einer Pixelfarbe von mehreren Pixeln der Videobilddaten eingefügt werden. Damit wird die Farbtiefe reduziert, weil nicht mehr die gesamten Bits für die Videobilddaten zur Verfügung stehen. Bspw. kann anstelle von 8 Bit pro Farbkanal (R, G, B) mit einer 24 Bit Nutzinformation pro Pixel die Farbtiefe auf 7 Bit pro Farbkanal reduziert werden, während die restlichen 3 Bits pro Pixel zur Speicherung oder Codierung der weiteren Daten verwendet werden. Es wird kein Teil des Bildes mehr komplett überblendet, sondern nur geringfügig in der Farbe angepasst. Um die hohe erforderlichen Qualität der intra-operativen Videobilder sicherzustellen, ist auch hier eine Speicherung der Daten in weniger relevanten Bildbereichen mit reduziertem Nutzinformationsgehalt, bspw. in Randbereichen, zu bevorzugen.

Da Videobilddaten für eine Archivierung in der Regel verlustbehaftet komprimiert werden müssen, um die Datenmenge geeignet zu reduzieren, ist vorzugsweise eine Repräsentation der Daten in den Videobildern zu wählen, die durch gängige Kompressionsverfahren, wie z.B. H.264, H.265, AV1 oder VP9, nicht so stark beeinflusst wird, dass die enthaltene Information nicht mehr rekonstruiert werden kann. Gemäß einer vorteilhaften Weiterbildung des erfindungsgemäßen Verfahrens werden die modifizierten Videobilddaten vor der Durchführung eines Kompressionsverfahrens mittels einer Prozedur zur Kompressionsinvarianzsteigerung aufgearbeitet, um die Zuverlässigkeit einer Rekonstruktion der Videobilddaten nach einer Dekompression unter Elimination oder deutlicher Reduktion von Codierungsfehlern oder Komprimierungsartefakten zu erhöhen.

Bspw. können die Messwerte oder sonstigen weiteren Daten in mehreren zusammenhängenden Pixeln, insbesondere unter Bildung von Pixelblöcken, z.B. 8x8, 16x16 oder sogar bis zu 64x64 Pixelblöcken, in jedem Einzelbild des Videostreams gespeichert werden. Derartige relativ gröbere Strukturen, die mehr Fläche im Bild belegen, neigen bei einer Komprimierung weniger zu Artefakten, so dass durch die Blockbildung die Robustheit gegen Kompressionsartefakte gesteigert wird.

Zusätzlich oder alternativ können auch gängige Fehlerkorrektur-Verfahren auf die weiteren Daten angewandt werden. Dies reduziert zwar weiter die effektive Datenmenge, erhöht aber zusätzlich die Zuverlässigkeit der Rekonstruktion unter Vermeidung von Kompressionsartefakten.

Vorzugsweise werden das Modifizieren der Videobilddaten und das Komprimieren der modifizierten Videobilddaten in einer gemeinsamen Signalverarbeitungseinrichtung durchgeführt. Die Signalverarbeitungseinrichtung kann dann die Prozedur zur Kompressionsinvarianzsteigerung in Abhängigkeit von dem gewählten Kompressionsverfahren durchführen und gegebenenfalls auch überprüfen, ob die Daten korrekt gespeichert werden können, oder die Strukturgröße der Daten dynamisch an die jeweilige Kompression anpassen.

In einer Weiterbildung des Verfahrens können ferner Schritte zur Auswertung der gespeicherten modifizierten Videobilddaten ausgeführt werden. Diese Schritte können ein Abrufen der gespeicherten modifizierten Videobilddaten, Extrahieren der weiteren Daten aus den modifizierten Videobilddaten und Analysieren der extrahierten statischen Daten und der gemessenen dynamischen Daten, insbesondere elektrischer Messdaten, in Verbindung mit den Videobilddaten zur Auswertung des durchgeführten medizinischen Eingriffs enthalten. Insbesondere kann die Auswertung eines oder mehrere der folgenden Auswertungsziele umfassen: Beurteilung der für den medizinischen Eingriff eingestellten Eingangsgrößen oder Parameter, Bewertung der gewählten Anwendungsmodi, Beurteilung der Wirksamkeit der gewünschten Behandlungseffekte, Auffinden von Optimierungsmöglichkeiten für die Einstellgrößen, Anwendungsmodi oder den oder die medizinischen Geräte, Erkennen von Unzulänglichkeiten, einschließlich elektrischer Grenzen für die Anwendung, z.B. eines für einen Schneidvorgang unzureichenden HF-Stroms oder einer unzureichenden HF-Spannung, Erkennen einer unzureichenden Geschwindigkeit der Behandlungseffekte, z.B. beim Schneiden, bei der Koagulation oder Thermofusion, Erfassung von Auftreten von Lateralschäden, wie z.B. Karbonisierung, Einsetzen einer Blutung, z.B. anhand der im Zeitverlauf gemessenen elektrischen Größen, und Ermittlung von Ursachen für die Lateralschäden oder andere Komplikationen. Diese Auswertungsziele können insbesondere durch eine Auswertung der dynamischen elektrischen Messdaten in Verbindung mit zugehörigen Bildinformationen erfolgen, wie z.B. einer visuell erkennbaren Funkenbildung und -intensität, einer im Bild erkennbaren Elektrode bzw. eines Instrumentes, eines im Bild erkennbaren Gewebes und/oder der Übergänge zwischen verschiedenen Gewebetypen, sichtbaren Blutungen oder einer sichtbaren Spülung mit Flüssigkeiten, einer sichtbaren Rauchentwicklung und/oder Dampfbildung und Wärmeentwicklung bei der Thermofusion.

Die Auswertung der vorstehend genannten Zusammenhänge kann manuell durch einen Bediener oder automatisch durch Verwendung eines geeigneten Algorithmus auf der Basis von Mustererkennung, Deep Learning, neuronalen Netzen oder auch künstlicher Intelligenz erfolgen. Die gewonnenen Erkenntnisse können für den behandelnden Chirurgen zur Qualitätssicherung, für künftige Anwender und für die Weiterentwicklung der medizinischen HF-Geräte nützlich sein.

Gemäß einem weiten Aspekt der Erfindung ist ein System zur Unterstützung einer Auswertung einer videounterstützen medizinischen Eingriffsprozedur geschaffen, wobei das System aufweist: wenigstens ein medizinisches Gerät zur Durchführung eines medizinischen Eingriffs, insbesondere ein HF-Chirurgiegerät; eine Videokamera zur Aufnahme von Videobilddaten, die Videobilder von einer behandelten oder untersuchten Anatomie während eines medizinischen Eingriffs repräsentieren; eine Signalverarbeitungseinrichtung zur Verarbeitung der von der Videokamera aufgenommenen Videobilddaten; und eine Datenverbindung zwischen dem wenigstens einen medizinischen Gerät und der Signalverarbeitungseinrichtung zur Übertragung von weiteren Daten, einschließlich dynamischer Daten, von dem wenigstens einen medizinischen Gerät zu der Signalverarbeitungseinrichtung. Die Signalverarbeitungseinrichtung ist zur Durchführung des Verfahrens, wie vorstehend beschrieben, eingerichtet.

Die Zusammenführung von Untersuchungs-, Geräte-, Diagnose- und/oder Messdaten mit den Videobilddaten findet somit in der Signalverarbeitungseinrichtung statt, die vorzugsweise auf einer von der medizinischen Behandlungs- oder Untersuchungsvorrichtung gesonderten dedizierten Rechenvorrichtung, z.B. einem Computer oder dgl., implementiert ist. Die Signalverarbeitungseinrichtung könnte auch in ein medizinisches Gerät integriert sein. Jedenfalls weist die Signalverarbeitungseinrichtung vorzugsweise Schnittstellen auf, um sie mit dem oder den medizinischen Geräten, einschließlich der weiteren Systemkomponenten und Instrumente und anderen Einrichtungen in dem Operationssaal, wie z.B. einem Patientenmonitor oder Operationstisch, zu verbinden.

Die Signalverarbeitungseinrichtung ist vorzugsweise dazu eingerichtet, das erfindungsgemäße Verfahren online, während der Durchführung des medizinischen Eingriffs, in Echtzeit oder nahezu in Echtzeit durchzuführen. Die Videobilddaten und weiteren Daten könnten aber prinzipiell auch offline und/oder nachträglich in der erfindungsgemäßen Weise durch die Signalverarbeitungseinrichtung verarbeitet werden, falls sie in geeigneter Weise, z.B. unter Verwendung von Zeitstempeln oder dgl., derart zwischengespeichert werden, dass eine nachträgliche zeitliche Zuordnung zur synchronen Zusammenführung der Video- und weiteren Daten möglich ist.

In einer Weiterbildung kann das erfindungsgemäße System auch für eine nachgelagerte Auswertung der Daten eingerichtet sein. Hierzu kann eine zusätzliche Soft- oder Firmware mit erweiterten Algorithmen bspw. in der Signalverarbeitungseinrichtung implementiert sein, die es ermöglichen, die weiteren Daten, insbesondere die statischen Behandlungs- oder Untersuchungs-, Gerätedaten und dynamischen Messwerte aus den einzelnen Frames zu extrahieren und diese für eine Analyse bereitzustellen oder die Daten mit einem Algorithmus automatisch auszuwerten.

Im Übrigen gelten die im Zusammenhang mit dem erfindungsgemäßen Verfahren oben gemachten Erläuterungen zu möglichen Ausführungsformen und deren Vorteilen in gleicher Weise für das erfindungsgemäße System, so dass auf diese ausdrücklich verwiesen wird.

Weitere vorteilhafte Einzelheiten von Ausführungsformen der Erfindung ergeben sich aus Unteransprüchen, der Zeichnung sowie der zugehörigen Beschreibung. In der Zeichnung sind keinesfalls beschränkende Ausführungsbeispiele des Gegenstandes der Erfindung dargestellt. Es zeigen:
Figur 1 eine Ausführungsform eines Systems zur Unterstützung einer Auswertung einer videounterstützten medizinischen Eingriffsprozedur gemäß einer Ausführungsform der Erfindung;
Figur 2 ein Flussdiagramm eines beispielhaften Verfahrens zur Unterstützung einer Auswertung einer videounterstützten medizinischen Eingriffsprozedur gemäß einer Ausführungsform der Erfindung;
Figuren 3a - 3c Darstellungen von Videoeinzelbildern aus einer medizinischen Eingriffsprozedur zur Veranschaulichung von Zielbereichen zur Einbettung von weiteren Daten in Videobilddaten, in einer stark vereinfachten Darstellung;
Figur 4 ein Prinzipschaubild zur Erläuterung der Funktions- oder Wirkungsweise des erfindungsgemäßen Systems und Verfahrens; und
Figur 5 ein Verfahren zur Unterstützung einer Auswertung einer videounterstützten medizinischen Eingriffsprozedur gemäß einer Weiterbildung der Erfindung.

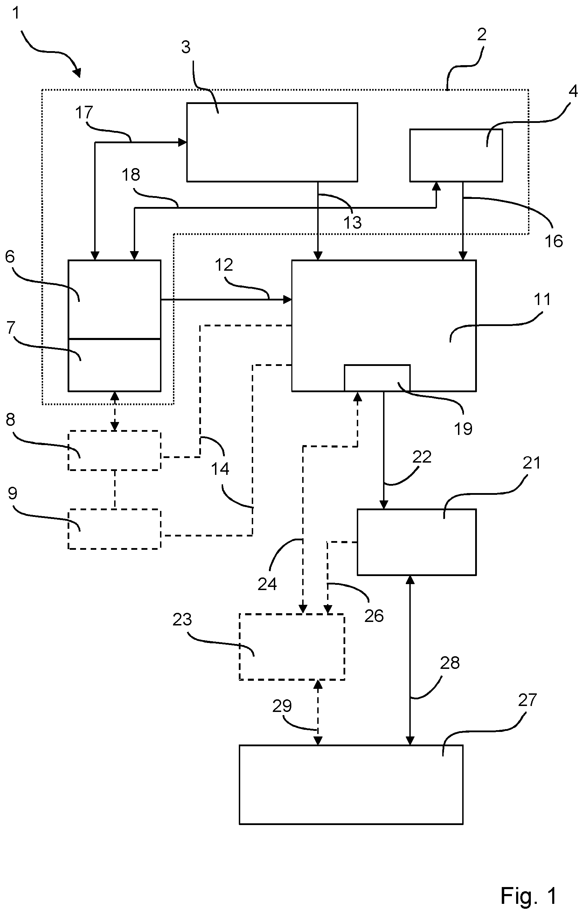

In Figur 1 ist in einer stark vereinfachten Blockbilddarstellung ein System 1 zur Unterstützung einer Auswertung einer videounterstützten medizinischen Eingriffsprozedur gemäß einer Ausführungsform der Erfindung dargestellt. Das System 1 enthält eine medizinische Behandlungs- und/oder Untersuchungsvorrichtung 2, die zur Untersuchung oder Behandlung, z.B. Therapie oder Operation, von Patienten eingerichtet ist. In bevorzugten Ausführungsformen ist die Vorrichtung 2 eine für die HF-Chirurgie verwendete HF-Operationsvorrichtung.

Die Behandlungs- und/oder Untersuchungsvorrichtung 2 weist ein medizinisches Gerät 3, insbesondere ein HF-Chirurgiegerät auf, das z.B. zum HF-Schneiden, zur Koagulation, Devitalisierung oder Thermofusion in der offenen Chirurgie oder für laparoskopische diagnostische oder therapeutische Verfahren oder ein endoskopisches HF-Gerät, bspw. zur Argon-Plasma-Koagulation, dient. Es sind noch andere medizinische Geräte, insbesondere Therapiegeräte, wie HF-, Kryo- oder Wasserstrahlgeräte, oder diagnostische Geräte, wie z.B. zur optischen Emissionsspektrometrie oder zur Impedanzspektroskopie denkbar, auf die die Erfindung anwendbar ist.

Die Behandlungs- und/oder Untersuchungsvorrichtung 2 weist ferner eine Videokamera 4 auf, die dazu eingerichtet ist, während eines medizinischen Eingriffs Videobilder einer untersuchten oder behandelten Anatomie mit einer vorbestimmten Framerate, wie z.B. mit 25 Frames pro Sekunde (fps), 30 fps, 50 fps oder 60 fps, zu erfassen. Je höher die Framerate (Bildwiederholfrequenz) ist, desto flüssiger können Videos abgespielt und Details auch bei hoher Dynamik in den Videoeinzelbildern oder im Zeitlupentempo scharf wiedergegeben werden.

Wenngleich die Videokamera 4 in dem in Fig. 1 dargestellten System 1 als von dem medizinischen Gerät 3 gesondert dargestellt ist, kann sie, z.B. bei endoskopischen Systemen, auch in das medizinische Gerät 3 integriert sein.

Die Behandlungs- und/oder Untersuchungsvorrichtung 2 weist ferner eine Steuerungsvorrichtung 6 auf, die mit dem medizinischen Gerät 3 verbunden ist, um den Betrieb des medizinischen Gerätes 3 zu steuern bzw. zu regeln. Insbesondere kann die Steuerungsvorrichtung 6 einen HF-Generator umfassen, um die für den Betrieb des medizinischen Gerätes 3 erforderlichen elektrischen Größen, wie HF-Spannungen und HF-Ströme, generieren, einstellen und an das medizinische Gerät 3 liefern zu können. Die Steuerungsvorrichtung 6 kann im Betrieb die Stärke der elektrischen Größen steuern oder regeln, die Modulationsfrequenz der elektrischen Größen dynamisch anpassen und andere Steuerungs- bzw. Regelungsoperationen durchführen, um das medizinische Gerät 3 zur Durchführung der jeweils gewünschten Untersuchungs- oder Behandlungsoperation zu veranlassen.

Die Steuerungsvorrichtung 6 kann mit einer hier nicht näher dargestellten Eingabevorrichtung verbunden sein, die dem Benutzer, bspw. einem behandelnden Arzt, ermöglicht, die gewünschten Einstellungen vorzunehmen oder Parameter auszuwählen. Vorzugsweise kann die Steuerungsvorrichtung einem Benutzer ermöglichen, aus mehreren vorgegebenen Modi auszuwählen, die für bestimmte Anwendungen vorbestimmt und auf bestimmte Arbeitsinstrumente abgestimmt sind, so z.B. verschiedene Schneid-, Koagulations- oder Gefäßversiegelungsmodi.

Die Steuerungsvorrichtung 6 kann auch die Zuführung von Gasen, wie z.B. Argon, oder Flüssigkeiten, bspw. zur Spülung von Gewebe, zu dem medizinischen Gerät 3 steuern.

Die Steuerungsvorrichtung 6 kann außerdem den Betrieb der Videokamera 4 steuern, wie dies anhand der dargestellten Kommunikationsverbindung zwischen der Videokamera 4 und der Steuerungsvorrichtung 6 in Fig. 1 angezeigt ist.

Die Steuerungsvorrichtung 6 kann auch zur Überwachung einer medizinischen Eingriffsprozedur eingerichtet sein und hierzu von dem medizinischen Gerät 3 Messdaten erhalten, die im Betrieb gemessenen oder ermittelten Werten entsprechen. Hierzu können an dem medizinischen Gerät 3 Sensoren vorgesehen sein, die die jeweiligen Größen, insbesondere elektrische Größen, während eines Betriebs des medizinischen Gerätes 3 erfassen. Zu den Messgrößen können elektrischen HF-Messgrößen, wie Spannung, Strom, Leistung, Leistungsfaktor oder Funkenintensität gehören, die während des Betriebs in situ an der Operationsstelle gemessen oder aus anderen Messwerten ermittelt werden. Zu den Messgrößen können bei monopolaren HF-Behandlungs- oder Untersuchungstechniken auch Parameter oder Größen einer Neutralelektrode, wie Übergangsimpedanz, Stromsymmetrie oder Stromdichte, gehören, die ebenfalls während des medizinischen Eingriffs sensorisch erfasst oder ermittelt werden können.

Die erfassten Daten können in einem nicht flüchtigen Speicher 7, bspw. einem Flash-Speicher der Steuerungsvorrichtung 6, zwischengespeichert werden.

Wenngleich die Steuerungsvorrichtung 6 mit dem Speicher 7 hier als von dem medizinischen Gerät 3 gesonderte Einheiten dargestellt sind, können sie wenigstens zum Teil auch in das medizinische Gerät 3 integriert sein. Es sind auch andere Konfigurationen für die Behandlungs- und/oder Untersuchungsvorrichtung 2 möglich, die von der in Fig. 1 dargestellten Konfiguration abweichen können.

Wie ferner aus Fig. 1 ersichtlich, kann das System 1 weitere medizinische Geräte 8, 9 aufweisen. Bspw. kann ein medizinisches Gerät 8 zur Durchführung einer anderen HF-Behandlung eingerichtet sein als das medizinische Gerät 3. Z.B. kann das medizinische Gerät 3 zur Koagulation oder Gefäßversiegelung eingerichtet sein, während das medizinische Gerät 8 zur Durchführung von Schneidoperationen vorbereitet und eingerichtet ist. Die Steuerungsvorrichtung 6 kann eingerichtet sein, um beide medizinische Geräte 3 und 8 während eines medizinischen Eingriffs abwechselnd zu steuern, zu regeln und/oder zu überwachen.

Ein noch weiteres medizinisches Gerät 9 kann eine weitere Systemkomponente, bspw. für einen Rauchabzug, ein anderes Instrument, eine andere Einrichtung in dem Operationssaal, wie z.B. einen Patientenmonitor, einen OP-Tisch und dgl. repräsentieren, die alle mit der Vorrichtung 2 vernetzt sein können. Die von den optionalen weiteren medizinischen Geräten 8, 9 gelieferten Daten können gemeinsam mit Daten von dem medizinischen Gerät 3 bzw. der Steuerungsvorrichtung 6 und Videobilddaten von der Videokamera 4 verknüpft und abgespeichert werden, um für eine nachträgliche Auswertung eines medizinischen Eingriffs zur Verfügung zu stehen.

Um dies zu bewerkstelligen, weist das System 1 ferner eine Signalverarbeitungseinrichtung 11 auf, die dazu eingerichtet ist, die von der Videokamera 4 aufgenommenen Videobilddaten und von dem medizinischen Gerät 3 bzw. der Steuerungsvorrichtung 6 gelieferte weitere Daten sowie gegebenenfalls von den weiteren medizinischen Geräten 8 und 9 bereitgestellte Daten zu verarbeiten, zeitlich synchron zusammenzuführen und in einer synchronisierten Weise abzuspeichern. Hierzu ist die Signalverarbeitungseinrichtung 11 über wenigstens eine Datenverbindung mit den anderen Systemkomponenten kommunikationsmäßig verbunden.

In der in Fig. 1 dargestellten beispielhaften Ausführungsform ist eine erste Datenverbindung 12 zwischen der Steuerungsvorrichtung 6 und der Signalverarbeitung 11 vorgesehen, um Daten, insbesondere Behandlungs- oder Untersuchungsdaten, Gerätedaten, Diagnose- und/oder Messdaten, die während einer medizinischen Eingriffsprozedur erfasst oder generiert und in dem internen Speicher 7 der Steuervorrichtung 6 zwischengespeichert werden können, von der Steuerungsvorrichtung 6 zu der Signalverarbeitungseinrichtung 11 zu übertragen. Optional kann zusätzlich eine zweite Datenverbindung 13 zwischen dem medizinischen Gerät 3 und der Signalverarbeitungseinrichtung 11 eingerichtet sein, um Daten direkt von dem medizinischen Gerät 3 zu der Signalverarbeitungseinrichtung 11 unter Umgehung der Steuerungsvorrichtung 6 übertragen zu können. Eine derartige Konfiguration könnte bei besonders zeitkritischen und mit hoher Abtastrate erfassten Sensordaten von Vorteil sein. Es können weitere Datenverbindungen 14 zwischen den weiteren medizinischen Geräten 8, 9 und der Signalverarbeitungseinrichtung 11 für gleiche Zwecke eingerichtet sein.

Außerdem ist in der Ausführungsform nach Fig. 1 eine Videodatenverbindung 16 zwischen der Videokamera 4 und der Signalverarbeitungseinrichtung 11 zur Übertragung von Videodaten von der Videokamera 4 zu der Signalverarbeitungseinrichtung 11 vorgesehen.

Die Datenverbindungen 12, 13, 14, 16 in dem System 1 könnten auf einem Bussystem basieren. Anstelle der gesonderten Verbindungen 12, 13 und 16 könnte auch eine einzige Datenverbindung 12 zwischen der Steuerungsvorrichtung 6 und der Signalverarbeitungseinrichtung 11 vorgesehen sein, und sämtliche Daten von dem medizinischen Gerät 3 und der Videokamera 4 könnten dann über Kommunikationsleitungen 17, 18 zuerst an die Steuervorrichtung 6 übermittelt und von dieser über die Datenverbindung 12 an die Signalverarbeitungseinrichtung 11 weitergeleitet werden.

Die Signalverarbeitungseinrichtung 11 weist ferner eine Datenschnittstelle 19 auf, die zur Datenkommunikation mit externen Einrichtungen und Geräten, wie bspw. einer externen Speichervorrichtung 21 eingerichtet ist. Die Speichervorrichtung 21 kann ein Server zur Archivierung von Bild-, Video-, Text- und sonstigen Daten, ein verteiltes Speichersystem, ein Cloud-basiertes Speichersystem oder ein sonstiger nichtflüchtiger Speicher sein, der in der Lage ist, die große Menge an Videobilddaten, die während einer medizinischen Eingriffsprozedur erzeugt werden, dauerhaft zu speichern. Die Speichervorrichtung 21 ist über eine Datenleitung 22 an die ausgangsseitige Datenschnittstelle 19 der Signalverarbeitungseinrichtung 11 angeschlossen und empfängt darüber von der Signalverarbeitungseinrichtung 11 modifizierte Videobilddaten, d.h. Videobilddaten mit darin eingebetteten weiteren Daten, zur Speicherung.

Wie ferner aus Fig. 1 ersichtlich, kann in einer Weiterbildung des erfindungsgemäßen Systems 1 die Signalverarbeitungseinrichtung 11 zusätzlich zu der Speichervorrichtung 21 oder alternativ dazu mit einem Krankenhausinformationssystem (KIS) kommunikationsmäßig verbunden sein, das informationsverarbeitende Systeme zur Erfassung, Bearbeitung und Weitergabe medizinischer und administrativer Daten in einem Krankenhaus umfasst. Somit können die von der Signalverarbeitungseinrichtung 11 erzeugten modifizierten Videobilddaten über die ausgangsseitige Datenschnittstelle 19 und eine Kommunikationsverbindung 24 zur weiteren Auswertung zu dem KIS 23 übertragen und dort gespeichert und bereitgestellt werden. Die Kommunikationsverbindung 24 kann eine drahtgebundene oder eine drahtlose Verbindung, bspw. eine LAN- oder WLAN-Verbindung, sein.

Alternativ kann das KIS 23 auch über eine Kommunikationsverbindung 26 mit der Speichervorrichtung 21 verbunden sein, um auf diese zugreifen und die relevanten Daten von dieser abrufen zu können.

Anstelle eines Krankenhausinformationssystems kann der Block 23 in Fig. 1 auch ein sog. Picture Archiving and Communication System (PACS) kennzeichnen, das ein in der Medizin weitgehend verbreitetes Bildarchivierungs- und Kommunikationssystem auf der Basis digitaler Rechner und Netzwerke darstellt. Mit einem PACS können digitale Bilddaten unterschiedlicher Modalitäten in der Radiologie, der Nuklearmedizin und auch Bilder aus anderen bildgebenden Verfahren, z.B. der Endoskopie, Kardiologie, etc. erfasst, verarbeitet und archiviert sowie bedarfsweise an weitere Betrachtungs- und Nachverarbeitungsrechner gesandt werden.

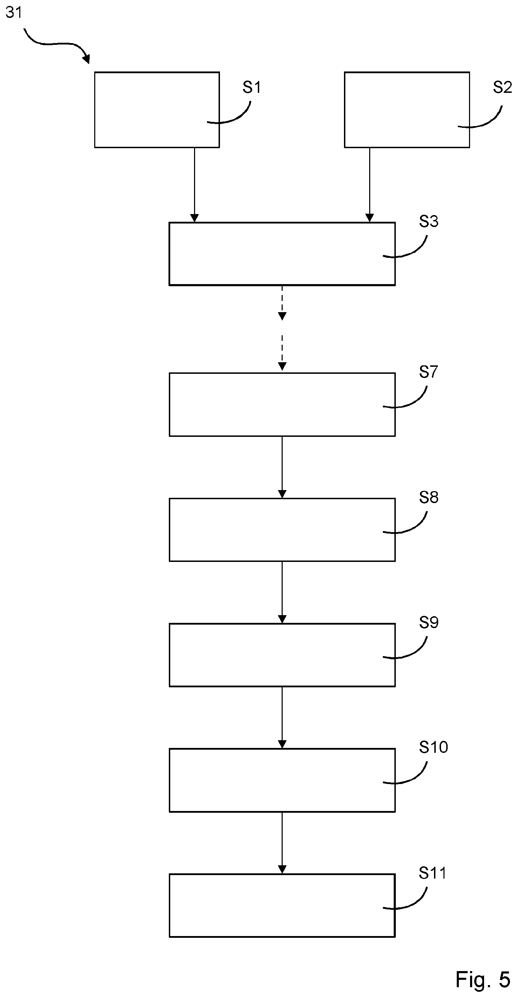

In einer noch weiteren Ausführungsform kann das System 1 ferner eine Analyseeinrichtung 27 umfassen, die über eine Datenübertragungsverbindung 28 mit der Speichervorrichtung 21 oder über eine Datenübertragungsverbindung 29 mit dem KIS oder PACS 23 verbunden sein kann, um auf das System 23 oder die Speichervorrichtung 21 zugreifen, Daten aus diesen auslesen und analysieren zu können. Insbesondere kann die Analyseeinrichtung 27 eine Soft- oder Firmware aufweisen, die dazu konfiguriert ist, aus den gespeicherten modifizierten Videobilddaten die weiteren Daten, wie bspw. Behandlungs- oder Untersuchungsdaten, Gerätedaten, Diagnosedaten und Messwerte, zu extrahieren, diese für eine Analyse durch einen Benutzer bereitzustellen oder diese automatisiert auf der Basis eines Algorithmus auszuwerten. Weitere Details zu den Funktionalitäten der Analyseeinrichtung 27 sind nachstehend im Zusammenhang mit dem erfindungsgemäßen Verfahren nach Fig. 5 angegeben.

Das soweit beschriebene System 1 dient zur Durchführung und Unterstützung einer Auswertung einer videounterstützten medizinischen Eingriffsprozedur. Der medizinische Eingriff kann insbesondere ein HF-Chirurgieeingriff, wie z.B. Koagulation, Thermofusion, HF-Schneiden (Elektrotomie) oder eine sonstige HF-Behandlung umfassen. Es kann auch eine Untersuchung bspw. mittels optischer Emissionsspektrometrie oder Impedanzspektroskopie umfassen, bei der neben Videobilddaten weitere Daten, insbesondere Diagnosedaten, generiert werden. Noch weiter könnte mit dem System 1 eine Therapiebehandlung, insbesondere eine HF-Therapie, durchgeführt werden. Das System 1 funktioniert wie folgt:

Während einer videounterstützten medizinischen Eingriffsprozedur erfasst die Videokamera 4 Videobilddaten, die Videobilder einer untersuchten oder behandelten Anatomie umfassen, mit einer bestimmten Framerate, wie bspw. 30, 50 oder 60 Frames pro Sekunde (fps) und überträgt die Videobilddaten zu der Signalverarbeitungseinrichtung 11 zur weiteren Verarbeitung. Außerdem überträgt die Steuerungsvorrichtung 6 und/oder das medizinische Gerät 3 weitere Daten zu der Signalverarbeitungseinrichtung 11. Diese weiteren Daten können Behandlungs- oder Untersuchungsdaten, Gerätedaten, Diagnosedaten und/oder Messdaten umfassen, die mit dem medizinischen Eingriff in Zusammenhang stehen. Insbesondere umfassen die weiteren Daten auch gemessene dynamische Daten, die sich während einer Frame-Dauer verändern.

Die Signalverarbeitungseinrichtung 11 empfängt die Videobilddaten von der Videokamera 4 und die weiteren Daten, einschließlich der dynamischen Messdaten, von der Steuerungsvorrichtung 6 und/oder dem medizinischen Gerät 3, identifiziert Zielbereiche in den Videobilddaten zur Einbettung der weiteren Daten und modifiziert die Videobilddaten durch zeitlich synchrones Einbetten der weiteren Daten in die Videobilddaten. Das heißt, die Signalverarbeitungseinrichtung 11 ersetzt Pixeldaten in den identifizierten Zielbereichen der Frames durch die zu dem jeweiligen Frame zugehörigen weiteren Daten.

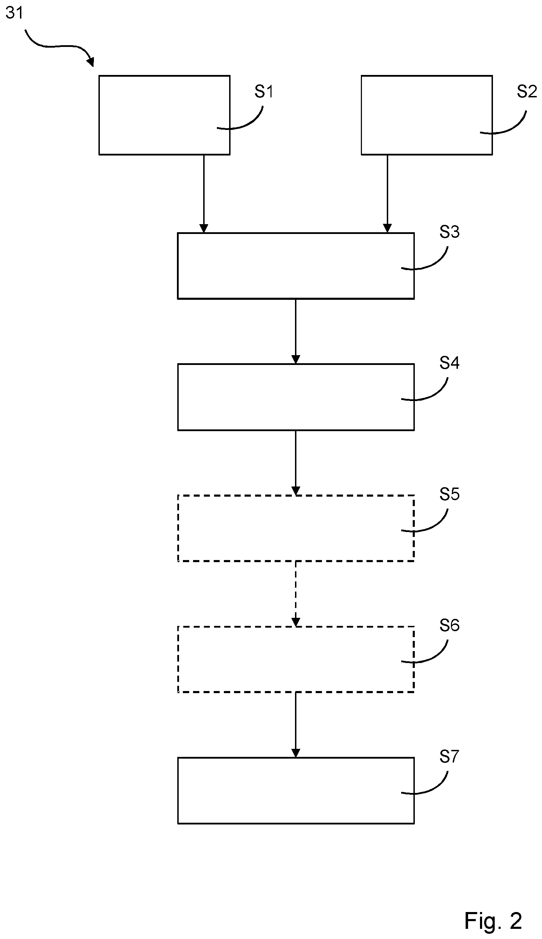

Weitere Details zu der Funktionsweise der Signalverarbeitungseinrichtung 11 im Zusammenhang mit dem zeitsynchronen Aufzeichnen von Videobilddaten und den weiteren Daten sind nachstehend im Zusammenhang mit dem erfindungsgemäßen Verfahren nach Figur 2 näher beschrieben.

Die Signalverarbeitungseinrichtung 11 kann die generierten modifizierten Videobilddaten über die Datenschnittstelle 29 an die Speichervorrichtung 21 und/oder das KIS oder PACS 23 zur kurz- bis langfristigen Archivierung übermitteln. Die Analyseeinrichtung 27 kann auf die archivierten modifizierten Videobilddaten zugreifen, um sie einer Analyse zu unterwerfen, um den durchgeführten medizinischen Eingriff und die zugehörige Arbeitsleistung der Behandlungs- und/oder Untersuchungsvorrichtung 2 auszuwerten.

Indem nun auf Figur 2 Bezug genommen wird, ist dort ein Flussdiagramm eines Verfahrens 31 zur Unterstützung einer Auswertung einer videounterstützten medizinischen Eingriffsprozedur gemäß einer Ausführungsform der vorliegenden Erfindung dargestellt. Das Verfahren kann von der Signalverarbeitungseinrichtung 11 des in Figur 1 dargestellten Systems 1 durchgeführt werden, ist jedoch nicht auf irgendeine spezielle Signalverarbeitungseinrichtung beschränkt. Es kann jede beliebige Recheneinheit, die einen Prozessor und einen Arbeitsspeicher aufweist, eingerichtet sein, um das Verfahren 31 nach Figur 2 auszuführen.

Das Verfahren 31 nach Figur 2 setzt voraus, dass ein videounterstützter medizinischer Eingriff durchgeführt wird (nicht veranschaulicht). Das Verfahren 31 kann dann online, während der Durchführung der videounterstützten medizinischen Eingriffsprozedur, in Echtzeit oder nahezu in Echtzeit ausgeführt werden. Es könnte auch nachträglich, nach der Durchführung der videounterstützten medizinischen Eingriffsprozedur ausgeführt werden, wenn alle Daten vorübergehend zwischengespeichert werden, obgleich dies weniger bevorzugt ist.

Das Verfahren 31 beginnt mit dem Schritt S1 des Empfangens von Videobilddaten. Bspw. kann die Signalverarbeitungseinrichtung 11 die Videobilddaten von der Videobildkamera 4 des Systems 1 nach Figur 1 empfangen. Wie erwähnt, werden die Videobilddaten mit einer bestimmten Framerate, vorzugsweise 30 fps, 50 fps oder 60 fps generiert, um eine gute optische Auflösung der Behandlungs- oder Untersuchungsvorgänge an der Anatomie in dem resultierenden Videostream zu ermöglichen.

Das Verfahren weist ferner den Schritt S2 des Empfangens weiterer Daten auf. Die weiteren Daten können eines oder mehrere von Behandlungs- oder Untersuchungs-, Geräte-, Diagnose- und/oder Messdaten umfassen, die mit dem medizinischen Eingriff im Zusammenhang stehen.

Bspw. können die weiteren Daten Behandlungs- oder Untersuchungsdaten umfassen, zu denen der Ort und die Zeit des Eingriffs, die behandelnde Institution, die beteiligten Personen, wie z.B. der Name des Patienten, Name des behandelnden Arztes, die Art des medizinischen Eingriffs und/oder andere den medizinischen Eingriff betreffende Daten gehören können.

Die weiteren Daten können insbesondere Gerätedaten umfassen, zu denen die Typenbezeichnung und/oder Seriennummer des oder der verwendeten medizinischen Geräte, vorgegebene Geräteparameter, Standardeinstellungen, gewählter Anwendungsmode, der automatisch bestimmte Geräteparametereinstellungen und Steuerung der elektrischen Größen bedingt, vorgegebene Grenzwerte für Variablen, bspw. die elektrischen Größen, Fehler- und Statusmeldungen, die durch die medizinischen Geräte während der medizinischen Eingriffsprozedur erzeugt werden, und andere, die verwendeten Geräte oder Instrumente betreffende Daten gehören können.

Zu den optional erfassten Diagnosedaten können während einer Untersuchung auf der Basis der optischen Emissionsspektrometrie ermittelte Spektren und/oder Klassifikationsergebnisse daraus gehören. Alternativ können auch Spektren, Messwerte und/oder Klassifikationsergebnisse aus der Impedanzspektroskopie oder aus anderen diagnostischen Verfahren empfangen und mit verarbeitet werden.

Zu den Messdaten, insbesondere den dynamischen Messdaten, können insbesondere elektrische HF-Größen wie Spannung, Strom, Leistung, Leistungsfaktor und/oder Funkenintensität gehören, die bspw. bei HF-Schneid- und HF-Koagulationsprozeduren erfasst oder ermittelt werden. Es können auch Parameter oder Größen einer Neutralelektrode bei Verwendung monopolarer HF-Techniken, wie z.B. die Übergangsimpedanz, Stromsymmetrie und/oder Stromdichte, etc., erfasst oder ermittelt und mit verarbeitet werden.

Das Verfahren 31 weist ferner den Schritt S3 des Identifizierens von Zielbereichen in den Videobildern zur Einbettung der weiteren Daten auf.

Moderne Videosysteme arbeiten mit einer Auflösung von FullHD (1920 x 1080 Pixel) oder 4k (4096 x 2160 Pixel) und einer Framerate von bspw. 30 fps. Wenn 0,1% der Pixel eines Videobildes zur Speicherung von Geräte-, Diagnose- und Messdaten genutzt werden, stehen ungefähr 2k Pixel pro Frame oder ca. 62k Pixel/s für FullHD und ca. 8,8k Pixel pro Frame oder ca. 265k Pixel/s für 4k zur Verfügung, um die weiteren Daten in die Videobilddaten einzubetten. Dies ergibt eine mögliche Datenmenge von ca. 182k Byte/s bei FullHD oder 778k Byte/s bei 4k. Es ist festgestellt worden, dass diese Datenmenge bereits bei FullHD ausreichend ist, um die weiteren Daten in den Videobilddaten zu speichern.

Zur Speicherung der Rohdaten von OES- oder Impedanzspektren wäre ein größerer Bildbereich von ca. 1% bei FullHD erforderlich, was grundsätzlich praktisch umsetzbar ist. Wenn statt Rohdaten nur die Ergebnisse der Klassifikation gespeichert werden, reicht eine deutlich geringere Datenmenge aus.

Prinzipiell können die Daten-Pixel zusammenhängend, **z.B.** als ein kleiner zusammenhängender Streifen oder Block, gespeichert werden, oder sie können an definierten Positionen über jedes Einzelbild verteilt werden. In dem Schritt S3 wird die geeignete Position oder werden die geeigneten Positionen zur Einbettung der Daten identifiziert. Die Identifizierung kann anhand im Voraus festgelegter oder durch einen Benutzer vorgegebener oder vorgebbarer geeigneter Bereiche in den Videobildern erfolgen. Alternativ könnte eine Kontexterkennungseinrichtung eingerichtet sein, um geeignete Bereiche auf dem darzustellenden Videobild zu identifizieren, die sich zur Einbettung der weiteren Daten in die Videobilddaten eignen.

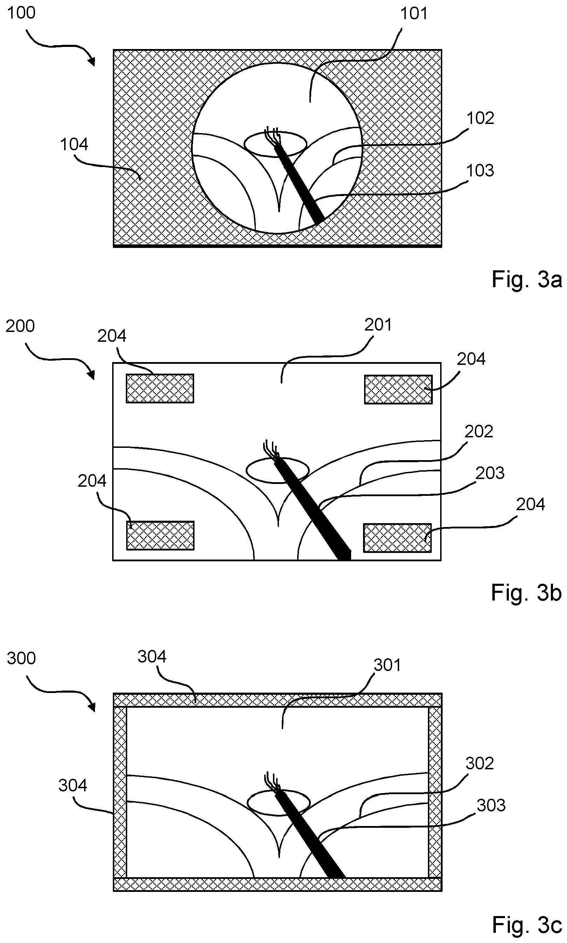

Figuren 3a-3c zeigen beispielhafte Videobilder 100, 200, 300, die Einzelbilder der Behandlungs- oder Untersuchungsstelle 101, 201, 301 mit einer darin veranschaulichten beispielhaften Anatomie 102, 202, 302 und einem Instrument 103, 203, 303 zur Behandlung der Anatomiestelle wiedergeben. Aus den Figuren 3a-3c ist erkennbar, dass als Zielbereiche zur Einbettung der weiteren Daten in den Videobildern abhängig von dem durchgeführten medizinischen Eingriff insbesondere Bereiche mit reduziertem Nutzinformationsgehalt, d.h. mit keinem oder geringem Informationsgehalt über die behandelte Anatomie 102, 202, 302, geeignet sind.

Z.B. zeigt Fig. 3a ein Einzelbild 101, das von einer endoskopischen Videokamera stammen kann und auf einen kreisrunden Bereich der Anzeige beschränkt sein kann. In diesem Fall ist zur Einbettung der weiteren Daten und Videobilddaten der gesamte das Einzelbild 101 umgebende Randbereich 104 geeignet.

Fig. 3b zeigt ein generiertes Videoeinzelbild 201, das im Wesentlichen die gesamte Anzeigefläche einer Anzeigevorrichtung einnimmt. Es sind reservierte Bereiche 204 dargestellt, die in der Nähe der Ecken des Videobildes 200 angeordnet und dazu bestimmt sind, Beschriftungen, Patientendaten, die Uhrzeit oder sonstige relevante Informationen bezüglich des medizinischen Eingriffs anzuzeigen. Ein oder mehrere derartige reservierte Bereiche 204 kann/können für die Einbettung der weiteren Daten ausgewählt werden.

Fig. 3c zeigt ein Beispiel, in dem der Bildrand 304 rings um das Einzelbild 301 als Zielbereich zur Einbettung der weiteren Daten verwendet werden kann.

In jedem Fall können die weiteren Daten in Form eines einzigen zusammenhängenden Streifens, Rechtecks oder dgl. an einer einzigen Position oder an mehreren über die Bereiche 104, 204, 304 verteilten Positionen gespeichert werden.

Zurückkehrend zu Fig. 2 ist veranschaulicht, dass das Verfahren 31 ferner den Schritt S4 des Modifizierens der Videobilddaten durch zeitlich synchrones Einbetten der weiteren Daten in die Videobilddaten aufweist. Insbesondere werden die Videobilddaten in den identifizierten Zielbereichen eines Frames durch die zu dem jeweiligen Frame zugehörigen weiteren Daten ersetzt. Das heißt, dass die modifizierten Videobilddaten in den jeweiligen Zielbereichen anstelle der ursprünglich erfassten Videobilddaten nun die weiteren Daten enthalten. Die weiteren Daten können als Rohdaten in das Videobild eingebettet oder alternativ vor der Einbettung verschlüsselt und/oder zur Sicherstellung der Authentizität mit einem digitalen Zertifikat versehen werden.

Ein wesentlicher Aspekt der Erfindung ist die zeitsynchrone Einbettung hochdynamischer Messdaten, z.B. der gemessenen elektrischen HF-Messwerte, die während einer medizinischen Eingriffsprozedur durch Sensoren erfasst oder ermittelt werden. Wie erwähnt, können diese z.B. die HF-Spannung, der HF-Strom, die Leistung, Leistungsfaktoren, Funkenintensitäten und andere Messwerte sein, die mit einer Aktualisierungsrate gemessen oder ermittelt werden, die um ein Mehrfaches, wenigstens Zweifaches oder sogar Vielfaches, größer ist als die Framerate der Videobilddaten. Die Aktualisierungsrate beträgt wenigstens 150 Hz und kann vorzugsweise 250 Hz oder mehr betragen. Jedenfalls werden diese hochdynamischen Messdaten schneller erfasst als die Framerate. Z.B. können bei einer Aktualisierungsrate von 250 Hz und einer Framerate von 50 Hz fünf Messwerte pro Frame gespeichert werden. Somit kann pro Einzelbild ein Zeitverlauf mit 5 oder mehreren Werten der dynamischen Messdaten aufgenommen und in den Videobilddaten zeitsynchron, jedem zugehörigen Frame zugeordnet abgespeichert werden.

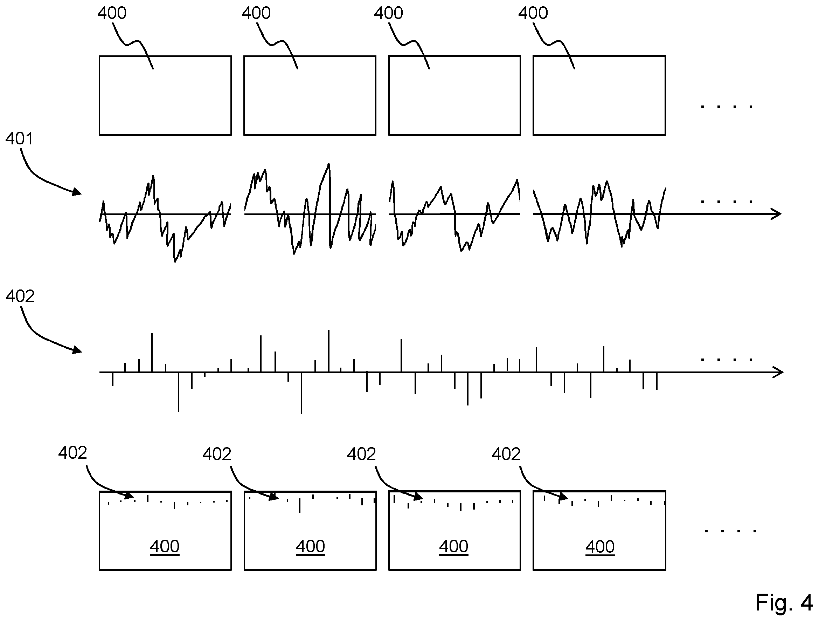

Fig. 4 veranschaulicht in einer vereinfachten Prinzipdarstellung die Einbettung dynamischer Daten in die einzelnen Videoframes. Die obere Grafik in Fig. 4 zeigt eine Folge von Videoframes 400, die aus den von einer Videokamera während einer medizinischen Eingriffsprozedur aufgenommenen Videobilddaten in einer Zeitsequenz resultieren.

Die zweitoberste Grafik der Fig. 4 zeigt eine beispielhafte elektrische HF-Größe 401, wie z.B. den HF-Strom oder die HF-Spannung, der bzw. die an das medizinische Gerät angelengt wird. Wie zu ersehen, ändert sich die elektrische Größe 401 so schnell, dass die Amplitude während einer Frame-Dauer hochfrequent schwankt.

Wie aus der drittobersten Grafik der Fig. 4 erkennbar, wird die elektrische Größe 401 mit einer Aktualisierungsrate gemessen oder ermittelt, die höher ist als die Framerate. Dadurch werden pro Frame-Dauer mehrere, **z.B.** fünf oder mehr, Messwerte 402 erhalten, die dem jeweiligen Videoframe 400 zugeordnet sind.

In der untersten Grafik in Fig. 4 ist veranschaulicht, wie die gewonnenen dynamischen Messdaten 403 in jeden Videoframe 400 integriert werden, um eine zeitlich synchrone Einbettung der dynamischen Messwerte 402 in die zugehörigen Videoframes 400 zu erhalten.

Es sollte erwähnt werden, dass in Fig. 1 die Einbettung der dynamischen Messwerte 402 in einem zusammenhängenden Bereich in einem oberen Randbereich der Videoframebilder nur beispielhaft ist. Es können verschiedene Bereiche unterschiedlicher Formen zur Speicherung der dynamischen Messwerte genutzt werden, wie vorstehend erläutert. Außerdem werden neben den dynamischen Messwerten auch andere Daten, z.B. Gerätedaten, mit in die einzelnen Frames 400 integriert.

Zurückkehrend zu Fig. 2 weist das Verfahren ferner den Schritt S7 der Abspeicherung der modifizierten Videobilddaten mit den in die Videobildframes eingebetteten weiteren Daten in einer Speichervorrichtung, bspw. der externen Speichervorrichtung 21 oder dem KIS oder PACS 23 in dem System nach Fig. 1, zur kurz- bis langfristigen Archivierung der Daten, um sie für eine spätere Analyse und Auswertung verfügbar zu machen. Das erfindungsgemäße Verfahren 31 kann dann nach dem Schritt S7 enden.

Im Allgemeinen werden die Videobilddaten im Schritt S7 in einer komprimierten Form gespeichert. Um eine möglichst geringe Datenmenge zu erhalten, werden in der Regel verlustbehaftete Komprimierungsalgorithmen, wie bspw. gängige Verfahren gemäß H.265, AV1 oder VP9, oder andere bekannte Kompressionsverfahren verwendet. Damit die weiteren Daten aus den abgespeicherten modifizierten Videobilddaten extrahiert und fehlerfrei rekonstruiert werden können, kann es sinnvoll sein, die erhaltenen modifizierten Videobilddaten im optionalen Schritt S5 einer Prozedur zur Kompressionsinvarianzsteigerung zu unterwerfen, die die Zuverlässigkeit einer Rekonstruktion der weiteren Daten nach einer Dekompression erhöht. Hierzu können die weiteren Daten bspw. zu mehreren zusammenhängenden Pixeln, insbesondere Pixelblöcken mit bspw. 8x8, 16x16 oder sogar bis zu 64x64 Pixeln, zusammengefasst werden. Es ist bekannt, dass derartige gröbere Strukturen in einem Bild bei einer Komprimierung weniger Kompressionsartefakte erleiden. Eine Blockbildung kann somit die Robustheit gegen Kompressionsartefakte und Kodierungsfehler steigern.

Als weitere Prozedur kann zusätzlich oder alternativ ein Fehlerkorrekturverfahren angewandt werden. Derartige Fehlerkorrekturverfahren, wie sie allgemein dazu dienen, Fehler bei der Speicherung und Übertragung von Daten zu erkennen und möglichst zu korrigieren, sind allgemein bekannt. Sie können hier zur Kodierung der weiteren Daten zur Einbettung in die Videobilddaten herangezogen werden, um einen nach der Dekompression vorliegenden Kodierungsfehler oder ein Kompressionsartefakt festzustellen und gegebenenfalls zu korrigieren.

Anschließend können die durch die Prozedur nach Schritt S5 aufgearbeiteten weiteren Daten in die Videobilddaten eingebettet und die resultierenden modifizierten Videobilddaten im Schritt S6 einer Kompression gemäß einem der vorstehend genannten gängigen Techniken unterworfen werden, um komprimierte modifizierte Videobilddaten zu erhalten, die anschließend im Schritt S7 abgespeichert werden. Es ist offensichtlich, dass die Aufarbeitung zur Steigerung der Kompressionsinvarianz nach Schritt S5 dem Schritt S4 zeitig vorgelagert oder nachgelagert seien oder auch gleichzeitig mit diesem erfolgen kann.

Außerdem ist von Vorteil, wenn die Schritte S4, S5 und S6 alle in einer gemeinsamen Signalverarbeitungsvorrichtung, wie bspw. der Signalverarbeitungseinrichtung 11 des Systems 1 nach Fig. 1, durchgeführt werden. Diese kann dann die Prozedur zur Kompressionsinvarianzsteigerung nach Schritt S5 in Abhängigkeit von dem gewählten Kompressionsverfahren durchführen und insbesondere vor der Abspeicherung der resultierenden Daten bereits überprüfen, ob die Daten korrekt gespeichert werden können bzw. ob die Strukturgröße der Daten an die Kompression dynamisch angepasst werden sollte, um eine korrekte Rekonstruktion der weiteren Daten zu ermöglichen.

Die Erfindung ermöglicht eine nachträgliche Analyse der aufgezeichneten Videobild- und sonstigen Daten, einschließlich hochdynamischer Messdaten, und ein Auswerten derselben zu unterschiedlichen Zielen. Insbesondere kann der durchgeführte medizinische Eingriff durch den behandelnden Arzt, das Krankenhaus oder den Patienten bedarfsweise nachverfolgt und bewertet werden, um den Erfolg oder Misserfolg des Eingriffs zu überprüfen. Die Analyse kann auch zur Beurteilung des Leistungsverhaltens des medizinischen Gerätes herangezogen werden. Bspw. können die für den medizinischen Eingriff eingestellten Eingangsgrößen, die gewählten Anwendungsmodi und die Wirksamkeiten der gewünschten Behandlungseffekte in allen Phasen des medizinischen Eingriffs durch Vergleich der Videobilddaten und der zugehörigen weiteren Daten beurteilt werden. Z.B. kann die Funkenbildung und -intensität visuell erfasst und mit zugehörigen elektrischen Messdaten verglichen werden. Somit können zeitkritische Vorgänge, wie bspw. das Zündverhalten oder ein Funkenabriss, anhand der eingebetteten elektrischen HF-Messdaten genau bewertet werden. Da diese Vorgänge viel schneller als eine Bildwiederholrate bzw. Framerate auftreten, ist die Erfassung der dynamischen elektrischen Messwerte im Zeitverlauf mit mehreren Messwerten pro Frame zur Bewertung derartiger zeitkritischer Vorgänge äußerst nützlich. Es können Optimierungsmöglichkeiten für die Einstellgrößen, Anwendungsmodi und das oder die medizinischen Geräte basierend auf den Auswerteergebnissen gesucht werden. Es können auch Unzulänglichkeiten, wie bspw. die Grenzen einer Einstellung für die jeweilige Anwendung, bspw. eine unzureichende Geschwindigkeit der Behandlungseffekte, z.B. beim Schneiden, Koagulieren, bei der Thermofusion und dgl., erkannt und mit den zugehörigen Einstellungen verglichen werden, um geeignetere Einstellungen zu finden. Es kann das Auftreten von Lateralschäden, wie Karbonisierung, Einsetzen einer Blutung, erfasst und anhand der erfassten Messdaten die Ursache für derartige Lateralschäden oder sonstige Komplikationen ermittelt werden.

Es lassen sich so viele weitere Auswertungen anhand der in den Videobilddaten eingebetteten weiteren Daten vornehmen, indem insbesondere die elektrischen Messdaten mit den visuell erfassbaren und in den Frames erkennbaren Elementen oder Ereignissen, wie Funkenbildung, Instrumente oder Elektroden, Gewebe und/oder Übergänge zwischen verschiedenen Gewebetypen, Blutungen oder Spülung mit Flüssigkeiten, Rauchentwicklung oder Dampfbildung und Wärmeentwicklung, verglichen werden. Die Auswertung der oben genannten Zusammenhänge kann manuell oder automatisch, durch einen geeigneten Algorithmus erfolgen.

Im Rahmen der Erfindung sind weitere Modifikationen möglich. Bspw. können die weiteren Daten, einschließlich der dynamischen Messdaten, anstatt in einzelne Pixel oder Pixelbereiche in einzelne Bits einer Pixelfarbe von mehreren Pixeln der Videobilddaten eingefügt werden. Wenn bspw. die Pixelfarbe jedes Pixels mit 24 Bit Nutzinformation kodiert wird (8 Bit pro Farbkanal (R,G,B)), kann die Farbtiefe z.B. auf 7 Bit pro Farbkanal reduziert werden, und die restlichen 3 Bits pro Pixel (1 Bit pro Farbkanal) können zur Speicherung oder Kodierung der weiteren Daten verwendet werden. Somit wird kein Teil des Bildes komplett überblendet, sondern nur geringfügig in der Farbe angepasst. Aufgrund der erforderlichen Qualität von intra-operativen Videos ist auch hier eine Anwendung in den weniger relevanten Bereichen, z.B. den Randbereichen oder den für Beschriftung, Patientendaten, Uhrzeit und dgl. reservierten Bereichen der Videobilder, zu bevorzugen.

Eine weitere Modifikation des erfindungsgemäßen Verfahrens 31 ist in Fig. 5 dargestellt. Die Schritte S1-S7 entsprechen dabei den Schritten in der Ausführungsform nach Fig. 2, so dass zur Vermeidung von Wiederholungen auf die dortigen Ausführungen verwiesen wird.

Wie aus Fig. 5 ersichtlich, weist das Verfahren 31 in der Ausführungsform nach Fig. 5 im Anschluss an den Schritt S7, in dem die modifizierten Videobilddaten abgespeichert werden, den Schritt S8 auf, in dem die modifizierten Videobilddaten aus dem jeweiligen Speicher, z.B. der Speichervorrichtung 21 oder dem KIS oder PACS 23 in dem System 1 nach Fig. 1, abgerufen werden.

Anschließend erfolgt im Schritt S9 eine Dekompression der Daten, um dekomprimierte Daten zu erhalten.

Aus den dekomprimierten Daten werden dann im Schritt S10 die ursprünglich in die Videobilddaten eingebetteten weiteren Daten, d.h. die gespeicherten Untersuchungs- oder Behandlungsdaten, Gerätedaten, Diagnosedaten und Messdaten, insbesondere dynamische Messdaten, extrahiert.

Die extrahierten weiteren Daten und die Videobilddaten werden im Schritt S11 einer Analyse mit einem oder mehreren der vorstehend erwähnten Ziele unterzogen, z.B. zur Qualitätsuntersuchung und -sicherung, zur Beurteilung der Einstellungen, Anwendungsmodi und Behandlungseffekte, zur Bewertung von Lateralschäden und sonstigen Komplikationen und zum Auffinden von Ursachen hierfür, zur Optimierung der Einstellungen oder Anwendungsmodi oder zur Geräteweiterentwicklung. Die Analyse kann manuell oder auch automatisiert erfolgen.

## Patentansprüche

1. Computer-implementiertes Verfahren zur Unterstützung einer Auswertung einer videounterstützen medizinischen Eingriffsprozedur, wobei das computer-implementierte Verfahren aufweist:
Empfangen von medizinischen Videobilddaten (S1), die während eines medizinischen Eingriffs von einer Videokamera (4) mit einer bestimmten Framerate aufgenommene Videobilder einer untersuchten oder behandelten Anatomie repräsentieren;
Empfangen von weiteren Daten (S2), die wenigstens eines von Behandlungs- oder Untersuchungs-, Geräte-, Diagnose- und Messdaten im Zusammenhang mit dem medizinischen Eingriff umfassen;
Identifizieren von Zielbereichen in den Videobildern zur Einbettung der weiteren Daten (S3); und
Modifizieren der Videobilddaten (S4) durch zeitlich synchrones Einbetten der weiteren Daten in die Videobilddaten, indem Videobilddaten in dem oder den identifizierten Zielbereichen eines Frames durch die zu dem jeweiligen Frame zugehörigen weiteren Daten ersetzt werden;
**dadurch gekennzeichnet, dass**
zu den weiteren Daten gemessene dynamische Daten gehören, die sich während einer Frame-Dauer verändern,
wobei zu den gemessenen dynamischen Daten Messgrößen gehören, die mit einer Aktualisierungsrate erfasst oder ermittelt werden, die um ein Mehrfaches größer ist als die Framerate der Videobilddaten, wobei die Aktualisierungsrate wenigstens 150 Hz beträgt, und
wobei in einzelnen Frames gemessene dynamische Daten gespeichert werden, die einen zeitlichen Verlauf von zwei oder mehreren Messwerten einer Messgröße kennzeichnen.

2. Computer-implementiertes Verfahren nach Anspruch 1, ferner mit wenigstens einem der folgenden Schritte:
Speichern der modifizierten Videobilddaten (S7) in einer nicht-flüchtigen Speichervorrichtung (21, 23) zur weiteren Verwendung, insbesondere Auswertung, in der Umgebung des Ortes der medizinischen Eingriffsprozedur; und/oder
Übertragung der modifizierten Videobilddaten zu einem Bildarchivierungs- und Kommunikationssystem (23), einem Krankenhausinformationssystem (23) und/oder einem externen Server (21) zur Archivierung von Bild-/Videodaten.

3. Computer-implementiertes Verfahren nach Anspruch 1 oder 2, wobei die Framerate zwischen 24 und 60 Frames pro Sekunde oder mehr, vorzugsweise 30, 50 oder 60 Frames pro Sekunde, beträgt.

4. Computer-implementiertes Verfahren nach einem beliebigen der vorhergehenden Ansprüche, wobei zu den Behandlungs- oder Untersuchungsdaten Ort und Zeit des Eingriffs, beteiligte Institution, beteiligte Personen, Name des Patienten, Name des behandelnden Arztes, Art des medizinischen Eingriffs und/oder andere den medizinischen Eingriff betreffende Daten gehören;
wobei zu den Gerätedaten eine Typenbezeichnung des oder der verwendeten medizinischen Geräte, Geräteseriennummer, Geräteparameter, Standardeinstellungen, gewählter Anwendungsmode, vorgegebene Grenzwerte für Variablen, Fehler- und Statusmeldungen der Geräte und/oder andere die verwendeten medizinischen Geräte betreffenden Daten gehören;
wobei zu den Diagnosedaten ermittelte Spektren und/oder Klassifikationsergebnisse aus einer optischen Emissionsspektrometrie, Spektren, Messwerte und/oder Klassifikationsergebnisse aus einer Impedanzspektroskopie oder aus anderen diagnostischen Verfahren gehören;
wobei zu den Messdaten gemessene oder ermittelte elektrische HF-Größen, wie Spannung, Strom, Leistung, Leistungsfaktor und/oder Funkenintensität, Parameter oder Größen einer Neutralelektrode, wie Übergangsimpedanz, Stromsymmetrie und/oder Stromdichte, und/oder andere während des medizinischen Eingriffs gemessene oder ermittelte Messgrößen gehören.

5. Computer-implementiertes Verfahren nach einem beliebigen der vorhergehenden Ansprüche, wobei zu den gemessenen dynamischen Daten Messgrößen gehören, die mit einer Aktualisierungsrate erfasst oder ermittelt werden, die 250 Hz oder mehr beträgt.

6. Computer-implementiertes Verfahren nach einem beliebigen der vorhergehenden Ansprüche, wobei in einzelnen Frames gemessene dynamische Daten gespeichert werden, die einen zeitlichen Verlauf von zwei oder mehreren Messwerten einer elektrischen Messgröße kennzeichnen.

7. Computer-implementiertes Verfahren nach einem beliebigen der vorhergehenden Ansprüche, wobei das Identifizieren von Zielbereichen in den Videobildern (S3) zur Einbettung der weiteren Daten anhand im Voraus festgelegter oder durch einen Benutzer vorgegebener oder vorgebbarer Bereiche in den Videobildern erfolgt.

8. Computer-implementiertes Verfahren nach einem beliebigen der vorhergehenden Ansprüche, wobei die weiteren Daten an definierten, über das Bild verteilten Positionen gespeichert werden.

9. Computer-implementiertes Verfahren nach einem beliebigen der vorhergehenden Ansprüche, wobei die weiteren Daten in Bereichen der Videobilder für Beschriftung, angezeigte Patientendaten, Uhrzeit und/oder am Bildrand eingebettet werden.

10. Computer-implementiertes Verfahren nach einem beliebigen der vorhergehenden Ansprüche, wobei die weiteren Daten in einzelne Bits einer Pixelfarbe von mehreren Pixeln der Videobilddaten, vorzugsweise in Bildbereichen mit geringerem Nutzinformationsgehalt, eingefügt werden.

11. Computer-implementiertes Verfahren nach einem beliebigen der vorhergehenden Ansprüche, wobei die modifizierten Videobilddaten verlustbehaftet komprimiert werden (S6), wobei die modifizierten Videobilddaten vor der Durchführung eines Kompressionsverfahrens mittels einer Prozedur zur Kompressionsinvarianzsteigerung aufgearbeitet werden (S5), um die Zuverlässigkeit einer Rekonstruktion der weiteren Daten nach einer Dekompression zu erhöhen.

12. Computer-implementiertes Verfahren nach Anspruch 11, wobei das Modifizieren der Videobilddaten (S4) und das Komprimieren der modifizierten Videobilddaten (S6) in einer gemeinsamen Signalverarbeitungsvorrichtung durchgeführt werden, die die Prozedur zur Kompressionsinvarianzsteigerung (S5) in Abhängigkeit von dem gewählten Kompressionsverfahren durchführt und vorzugsweise die Strukturgröße und/oder Codierung der Daten dynamisch an die Kompression anpassen kann.

13. Computer-implementiertes Verfahren nach einem der Ansprüche 2-12, ferner mit den Schritten:
Abrufen der gespeicherten modifizierten Videobilddaten (S8);
Extrahieren der weiteren Daten aus den modifizierten Videobilddaten (S10); und
Analysieren der extrahierten statischen und gemessenen dynamischen Daten (S11), insbesondere elektrischer Messdaten, in Verbindung mit den Videobilddaten zur Auswertung des durchgeführten medizinischen Eingriffs, wobei die Auswertung eines oder mehrere der folgenden Ziele umfasst: Beurteilung der für den medizinischen Eingriff eingestellten Eingangsgrößen, gewählten Anwendungsmodi und/oder der Wirksamkeit der gewünschten Behandlungseffekte, Auffinden von Optimierungsmöglichkeiten für die Einstellgrößen, Anwendungsmodi oder den oder die medizinischen Geräte, Erkennen von Unzulänglichkeiten, einschließlich elektrischer Grenzen für die Anwendung, unzureichender Geschwindigkeit der Behandlungseffekte, Erfassen von Auftreten von Lateralschäden, wie Karbonisierung, Einsetzen einer Blutung, und Ermitteln von Ursachen für die Lateralschäden oder andere Komplikationen.

14. System (1) zur Unterstützung einer Auswertung einer videounterstützen medizinischen Eingriffsprozedur, wobei das System (1) aufweist:
wenigstens ein medizinisches Gerät (3, 8, 9) zur Durchführung eines medizinischen Eingriffs, insbesondere ein HF-Chirurgiegerät;
eine Videokamera (4) zur Aufnahme von Videobilddaten, die Videobilder von einer behandelten oder untersuchten Anatomie während eines medizinischen Eingriffs repräsentieren;
eine Signalverarbeitungseinrichtung (11) zur Verarbeitung der von der Videokamera (4) aufgenommenen Videobilddaten;
eine Datenverbindung (12, 13, 14) zwischen dem wenigstens einen medizinischen Gerät (3, 8, 9) und der Signalverarbeitungseinrichtung (11) zur Übertragung von weiteren Daten, einschließlich dynamischer Daten, von dem wenigstens einen medizinischen Gerät (3, 8, 9) zu der Signalverarbeitungseinrichtung (11);
wobei die Signalverarbeitungseinrichtung (11) zur Durchführung des computer-implementierten Verfahrens nach einem der vorhergehenden Ansprüche eingerichtet ist.

15. System nach Anspruch 14, wobei die Signalverarbeitungseinrichtung (11) eine Datenschnittstelle (19) aufweist, die mit einem Bildarchivierungs- und Kommunikationssystem oder einem Krankenhausinformationssystem (23) und/oder einem externen Server (21) zur Archivierung von Bild-/Videodaten in Kommunikationsverbindung steht.

## Claims

1. A computer-implemented method for supporting evaluation of a video-assisted medical interventional procedure, the computer-implemented method comprising:
receiving medical video image data (S1) representing video images of an examined or treated anatomy recorded by a video camera (4) at a specific frame rate during a medical intervention;
receiving further data (S2) comprising at least one of treatment or examination, device, diagnostic and measurement data associated with the medical intervention;
identifying target areas in the video images for embedding the further data (S3); and
modifying the video image data (S4) by embedding the further data into the video image data synchronously in time by replacing video image data in the identified target area or areas of a frame with the further data associated with the respective frame;
**characterized in that**
the further data includes measured dynamic data that varies during a frame period,
the measured dynamic data includes measurement variables that are acquired or determined at an update rate that is several times greater than the frame rate of the video image data, with the update rate being at least 150 Hz, and
wherein measured dynamic data which characterizes a time course of two or more measured values of a measured variable is stored in individual frames.

2. The computer-implemented method of claim 1, further comprising at least one of the following steps:
storing the modified video image data (S7) in a nonvolatile storage device (21, 23) for further use, in particular evaluation, in the vicinity of the place of the medical interventional procedure; and/or
transmitting the modified video image data to a picture archiving and communication system (23), a hospital information system (23) and/or an external server (21) for archiving image/video data.

3. The computer-implemented method according to claim 1 or 2, wherein the frame rate is between 24 and 60 frames per second or more, preferably 30, 50 or 60 frames per second.

4. The computer-implemented method according to anyone of the preceding claims, wherein the treatment or examination data include place and time of the intervention, institution involved, persons involved, name of the patient, name of the attending physician, type of medical intervention and/or other data relating to the medical intervention;
wherein the device data includes a type designation of the medical device or devices used, device serial number, device parameters, default settings, selected application mode, preset limit values for variables, error and status messages from the devices and/or other data relating to the medical devices used;
wherein the diagnostic data includes determined spectra and/or classification results from an optical emission spectrometry, spectra, measured values and/or classification results from an impedance spectroscopy or from other diagnostic methods;
wherein the measurement data includes measured or determined electrical RF variables, such as voltage, current, power, power factor and/or spark intensity, parameters or variables of a neutral electrode, such as transition impedance, current symmetry and/or current density, and/or other measurement variables measured or determined during the medical intervention.

5. The computer-implemented method according to anyone of the preceding claims, wherein the measured dynamic data includes measurement variables that are acquired or determined at an update rate that is 250 Hz or more.

6. The computer-implemented method according to anyone of the preceding claims, wherein measured dynamic data which characterizes a time course of two or more measured values of a measured electrical variable is stored in individual frames.

7. The computer-implemented method according to anyone of the preceding claims, wherein identifying target areas in the video images (S3) for embedding the further data is based on areas in the video images that are defined in advance or are specified or can be specified by a user.

8. The computer-implemented method according to anyone of the preceding claims, wherein the further data is stored at defined positions distributed over the image.

9. The computer-implemented method according to anyone of the preceding claims, wherein the further data is embedded in areas of the video images for labeling, displayed patient data, time and/or at an image edge.

10. The computer-implemented method according to anyone of the preceding claims, wherein the further data is incorporated into individual bits of a pixel color of a plurality of pixels of the video image data, preferably in image areas with a lower useful information content.

11. The computer-implemented method according to anyone of the preceding claims, wherein the modified video image data is lossy compressed (S6), wherein the modified video image data is processed by a compression invariance enhancement procedure (S5) before a compression method is performed, in order to increase reliability of a reconstruction of the further data.

12. The computer-implemented method according to claim 11, wherein the modification of the video image data (S4) and the compression of the modified video image data (S6) are performed in a common signal processing device, which performs the compression invariance enhancement procedure (S5) depending on the selected compression method and is preferably able to dynamically adapt the structure size and/or encoding of the data to the compression.

13. The computer-implemented method according to anyone of the claims 2-12, further comprising the steps of:
retrieving the stored modified video image data (S8);
extracting the further data from the modified video image data (S10); and
analyzing the extracted static and measured dynamic data (S11), in particular electrical measurement data, in conjunction with the video image data for evaluating the medical intervention performed, the evaluation comprising one or more of the following goals: assessment of the input variables set for the medical intervention, the selected application modes and/or the effectiveness of the desired treatment effects, finding optimization possibilities for the set variables, application modes or the medical device or devices, detection of inadequacies, including electrical limitations for the application, insufficient speed of the treatment effects, detection of occurrence of lateral damage, such as carbonization, onset of bleeding, and determination of causes of the lateral damage or other complications.

14. System (1) for supporting evaluation of a video-assisted medical interventional procedure, the system (1) comprising:
at least one medical device (3, 8, 9) for performing a medical intervention, in particular an RF surgical device;
a video camera (4) for recording video image data representing video images of treated or examined anatomy during a medical intervention;
a signal processing device (11) for processing the video image data recorded by the video camera (4);
a data connection (12, 13, 14) between the at least one medical device (3, 8, 9) and the signal processing device (11) for transmitting further data, including dynamic data, from the at least one medical device (3, 8, 9) to the signal processing device (11);
wherein the signal processing device (11) is configured to perform the computer-implemented method according to one of the preceding claims.

15. The system according to claim 14, wherein the signal processing device (11) comprises a data interface (19) which is in communication with a picture archiving and communication system or a hospital information system (23) and/or an external server (21) for archiving image/video data.

## Revendications

1. Procédé mis en œuvre par ordinateur pour assister une évaluation d'une procédure d'intervention médicale assistée par vidéo, dans lequel le procédé mis en œuvre par ordinateur présente :
la réception de données d'image vidéo médicale (S1) qui représentent des images vidéo d'une anatomie étudiée ou traitée capturées par une caméra vidéo (4) à une fréquence d'images donnée pendant une intervention médicale ;
la réception d'autres données (S2) qui comprennent au moins l'une parmi des données de traitement ou d'examen, d'appareil, de diagnostic et de mesure liées à l'intervention médicale ;
l'identification de zones cibles dans les images vidéo pour intégrer les autres données (S3) ; et
la modification des données d'image vidéo (S4) par intégration synchrone dans le temps des autres données dans les données d'image vidéo du fait que les données d'image vidéo dans la ou les régions cibles identifiées d'une trame sont remplacées par les autres données associées à la trame concernée ;
**caractérisé en ce que**
les autres données incluent des données dynamiques mesurées qui changent pendant une durée de trame,
dans lequel les données dynamiques mesurées incluent des grandeurs de mesure qui sont acquises ou déterminées à une fréquence de rafraîchissement qui est un multiple plus élevée que la fréquence d'images des données d'image vidéo, dans lequel la fréquence de rafraîchissement est d'au moins 150 Hz, et
dans lequel des données dynamiques mesurées sont stockées dans des trames individuelles, qui caractérisent une variation dans le temps de deux valeurs de mesure ou plus d'une grandeur de mesure.

2. Procédé mis en œuvre par ordinateur selon la revendication 1, avec en outre au moins l'une des étapes suivantes :
le stockage des données d'image vidéo modifiées (S7) dans un dispositif de stockage non volatil (21, 23) pour une utilisation ultérieure, en particulier une évaluation, dans l'environnement du lieu de la procédure d'intervention médicale ; et/ou
le transfert des données d'image vidéo modifiées vers un système d'archivage et de communication d'images (23), un système d'information hospitalier (23) et/ou un serveur externe (21) pour l'archivage des données d'image/vidéo.

3. Procédé mis en œuvre par ordinateur selon la revendication 1 ou 2, dans lequel la fréquence d'images est comprise entre 24 et 60 images par seconde ou plus, de préférence 30, 50 ou 60 images par seconde.

4. Procédé mis en œuvre par ordinateur selon l'une quelconque des revendications précédentes, dans lequel les données de traitement ou d'examen incluent le lieu et l'heure de l'intervention, l'établissement impliqué, les personnes impliquées, le nom du patient, le nom du médecin traitant, le type d'intervention médicale et/ou d'autres données relatives à l'intervention médicale ;
dans lequel les données d'appareil incluent une désignation de type du ou des appareils médicaux utilisés, le numéro de série de l'appareil, les paramètres de l'appareil, les réglages par défaut, le mode d'application sélectionné, les valeurs limites prédéfinies pour les variables, les messages d'erreur et d'état des appareils et/ou d'autres données concernant les appareils médicaux utilisés ;
dans lequel les données de diagnostic incluent des spectres et/ou résultats de classification déterminés à partir d'une spectrométrie d'émission optique, des spectres, des valeurs de mesure et/ou des résultats de classification à partir d'une spectroscopie d'impédance ou d'autres procédés de diagnostic ;
dans lequel les grandeurs électriques HF mesurées ou déterminées pour les données de mesure, telles que la tension, le courant, la puissance, le facteur de puissance et/ou l'intensité de l'étincelle, paramètres ou grandeurs d'une électrode neutre, tels que l'impédance de transition, l'équilibre du courant et/ou la densité du courant, et/ou d'autres grandeurs de mesure mesurées ou déterminées pendant l'intervention médicale.

5. Procédé mis en œuvre par ordinateur selon l'une quelconque des revendications précédentes, dans lequel les données dynamiques mesurées incluent des grandeurs de mesure qui sont acquises ou déterminées à une fréquence de rafraîchissement qui est de 250 Hz ou plus.

6. Procédé mis en œuvre par ordinateur selon l'une quelconque des revendications précédentes, dans lequel des données dynamiques mesurées dans des trames individuelles sont stockées, qui caractérisent une variation dans le temps de deux valeurs de mesure ou plus d'une grandeur de mesure électrique.

7. Procédé mis en œuvre par ordinateur selon l'une quelconque des revendications précédentes, dans lequel l'identification de zones cibles dans les images vidéo (S3) pour intégrer les autres données est effectuée à partir de zones fixées d'avance ou prédéfinies ou pouvant être prédéfinies par un utilisateur dans les images vidéo.

8. Procédé mis en œuvre par ordinateur selon l'une quelconque des revendications précédentes, dans lequel les autres données sont stockées à des positions données réparties sur l'image.

9. Procédé mis en œuvre par ordinateur selon l'une quelconque des revendications précédentes, dans lequel les autres données sont intégrées dans des zones des images vidéo pour l'étiquetage, les données de patient affichées, l'heure et/ou sur le bord de l'image.

10. Procédé mis en œuvre par ordinateur selon l'une quelconque des revendications précédentes, dans lequel les autres données sont insérées dans des bits individuels d'une couleur de pixel de plusieurs pixels des données d'image vidéo, de préférence dans des zones d'image à faible teneur en informations utiles.

11. Procédé mis en œuvre par ordinateur selon l'une quelconque des revendications précédentes, dans lequel les données d'image vidéo modifiées sont compressées avec perte (S6), dans lequel les données d'image vidéo modifiées sont traitées avant la réalisation d'un procédé de compression au moyen d'une procédure d'augmentation d'invariance de compression (S5) pour augmenter la fiabilité d'une reconstruction des autres données après une décompression.

12. Procédé mis en œuvre par ordinateur selon la revendication 11, dans lequel la modification des données d'image vidéo (S4) et la compression des données d'image vidéo modifiées (S6) sont réalisées dans un dispositif de traitement de signal commun qui réalise la procédure d'augmentation d'invariance de compression (S5) en fonction du procédé de compression choisi et peut de préférence adapter dynamiquement la taille de structure et/ou le codage des données à la compression.

13. Procédé mis en œuvre par ordinateur selon l'une quelconque des revendications 2 à 12, en outre avec les étapes :
de rappel des données d'image vidéo modifiées stockées (S8) ;
d'extraction des autres données à partir des données d'image vidéo modifiées (S10) ; et
d'analyse des données dynamiques statiques et mesurées extraites (S11), en particulier des données de mesure électrique, en lien avec les données d'image vidéo pour évaluer l'intervention médicale réalisée, dans lequel l'évaluation comprend un ou plusieurs des objectifs suivants : l'appréciation des grandeurs d'entrée réglées pour l'intervention médicale, modes d'application choisis et/ou de l'efficacité des effets thérapeutiques souhaités, la recherche de possibilités d'optimisation pour les grandeurs de réglage, modes d'application ou le ou les appareils médicaux, le repérage des insuffisances, y compris les limites électriques pour l'application, la vitesse insuffisante des effets thérapeutiques, la détection de l'apparition de dommages latéraux, comme la carbonisation, la survenance d'une hémorragie, et l'identification des causes des lésions latérales ou d'autres complications.

14. Système (1) pour assister une évaluation d'une procédure d'intervention médicale assistée par vidéo, dans lequel le système (1) présente :
au moins un appareil médical (3, 8, 9) pour réaliser une intervention médicale, en particulier un appareil chirurgical HF ;
une caméra vidéo (4) pour enregistrer des données d'image vidéo qui représentent des images vidéo d'une anatomie traitée ou examinée pendant une intervention médicale ;
un équipement de traitement de signal (11) pour traiter les données d'image vidéo capturées par la caméra vidéo (4) ;
une liaison de données (12, 13, 14) entre l'au moins un appareil médical (3, 8, 9) et l'équipement de traitement de signal (11) pour transmettre d'autres données, y compris des données dynamiques, de l'au moins un appareil médical (3, 8, 9) à l'équipement de traitement de signal (11) ;
dans lequel l'équipement de traitement de signal (11) est configuré pour réaliser le procédé mis en œuvre par ordinateur selon l'une quelconque des revendications précédentes.

15. Système selon la revendication 14, dans lequel l'équipement de traitement de signal (11) présente une interface de données (19) qui est en liaison de communication avec un système d'archivage et de communication d'images ou un système d'information hospitalier (23) et/ou un serveur externe (21) pour l'archivage de données image/vidéo.
